# EUROPEAN PATENT APPLICATION

(11) **EP 4 640 712 A1**
(43) Date of publication of application: **29.10.2025**
(21) Application number: 23910772.5
(22) Date of filing: 27.12.2023
(51) Int. Cl.: C07K 16/28, C07K 16/46, C12N 15/13, C12N 15/85, A61K 39/395, A61P 35/00

(54) **ANTI-PDL1 ANTIBODY AND USE THEREOF**

(30) Priority: 31.12.2022 CN 202211737491
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Studio Torta S.p.A.
(86) International application number: PCT/CN2023/142507
(87) International publication number: WO 2024/140854

(57) **Abstract**

The present disclosure relates to the field of biomedicine, and more particularly, to an anti-PD-L1 antibody or antigen binding fragment and use thereof. The anti-PD-L1 antibody or antigen binding fragment according to the present disclosure includes a CDR selected from at least one of the following sequences or amino acid sequences having at least 80% identity thereto: heavy chain variable region CDR sequences: SEQ ID NO: 1, SEQ ID NO: 2, or SEQ ID NO: 3; or light chain variable region CDR sequences: SEQ ID NO: 4, WAS, or SEQ ID NO: 5. The antibody has a high binding affinity to PD-L1, and the present disclosure also provides a CD3 and PD-L1 bispecific antibody that has a stronger binding to tumor cells and promotes T cells to exert anti-cancer function.

## Description

### PRIORITY INFORMATION

This application claims the priority and benefit of Chinese Patent Application No. 202211737491.9 filed with the China National Intellectual Property Administration on December 31, 2022, which is incorporated herein by reference in its entirety.

### FIELD

The present disclosure relates to the field of biomedicine, and more particularly, to an anti-PD-L1 antibody or its antigen binding fragment and use thereof.

### BACKGROUND

Globally, cancer remains a major disease that threatens human life and health. With the worsening of environmental pollution, the incidence of cancer continues to rise year by year. Meanwhile, a variety of cancer therapies have been developed, including conventional approaches such as surgical resection, radiotherapy, and chemotherapy, as well as more recently emerging targeted therapies such as small molecule targeted drugs, large molecule targeted drugs, immune checkpoint inhibitors, bispecific antibodies, and cell and gene therapy.

In recent years, the most promising therapies have included immune checkpoint inhibitors and bispecific antibody drugs. With the approval of CTLA4 antibodies, PD-1/L1 antibodies, and LAG3 antibodies, immune checkpoint therapies have become a hot trend in recent years and have demonstrated remarkable efficacy in various tumors, including melanoma and non-small cell lung cancer. The approval of bispecific antibody drugs is also accelerating. To date, Eemovab (CD3×EPCAM), CD3×CD19 (Blincyto), Rybrevant (EGFR×cMet), Lunsumio (CD3×CD20), and Tecvayli (CD3×BCMA) have all been approved. Notably, Rybrevant (EGFR×cMet), Lunsumio (CD3×CD20), and Tecvayli (CD3×BCMA) were approved in the past two years, indicating that these innovative therapies are indeed bringing real benefits to more patients.

Radiotherapy and chemotherapy are generally accompanied by severe side effects and immunosuppression in patients, which may contribute to tumor recurrence. Unlike the cytotoxic mechanisms of conventional radiotherapy and chemotherapy, immune checkpoint therapy and bispecific antibody drugs activate the patient's own immune system to fight cancer, with fewer side effects and no compromised immune function in patients.

Programmed death-ligand 1 (PD-L1) is significantly upregulated in a variety of tumors. PD-L1 can bind to the programmed death-1 (PD-1) receptor on the surface of T cells, delivering immunosuppressive signals that promotes T cell exhaustion and impairs the patient's anti-cancer immunity. Antibodies that block the PD-1/PD-L1 interaction can reverse T cell exhaustion and restore anti-cancer immunity. To date, several PD-1 antibodies including Opdivo and Keytruda, as well as PD-L1 antibodies including Tecentirq (Atezolizumab) have demonstrated clinical benefit in a large number of patients. However, it should be noted that not all patients respond to PD-1/PD-L1 monoclonal antibody therapy, and the overall response rate is around 30%, leaving a substantial proportion of patients unresponsive to such treatment.

The extracellular domain of the PD-1 receptor or its variants is subject to heterogeneous glycosylation, which limits its developability as a therapeutic target. Although anti-PD-L1 antibodies and related products have been reported, they all exhibit insufficient binding affinity to PD-L1 expressed on the surface of tumor cells.

Therefore, it is urgently needed to develop an anti-PD-L1 antibody with enhanced binding affinity to PD-L1.

### SUMMARY

The present disclosure aims to solve, at least to some extent, one of the technical problems in the related art.

To this end, in a first aspect, the present disclosure provides an antibody or antigen binding fragment, which includes: a CDR selected from at least one of the following sequences or amino acid sequences having at least 80% identity thereto: heavy chain variable region CDR sequences: SEQ ID NO: 1, SEQ ID NO: 2, or SEQ ID NO: 3; or light chain variable region CDR sequences: SEQ ID NO: 4, WAS, or SEQ ID NO: 5.

The antibody or antigen binding fragment according to the present disclosure contains the above-mentioned specific CDR sequence, which can specifically bind to PD-L1 highly expressed on the surface of tumor cells with high binding affinity, thereby blocking the interaction between PD-1 and PD-L1 on the surface of the immune cells and promoting the anti-cancer function of the immune cells.

In some embodiments of the present disclosure, the antibody or antigen binding fragment includes: heavy chain variable region CDR1 with a sequence as set forth in SEQ ID NO: 1, heavy chain variable region CDR2 with a sequence as set forth in SEQ ID NO: 2, heavy chain variable region CDR3 with a sequence as set forth in SEQ ID NO: 3, light chain variable region CDR1 with a sequence as set forth in SEQ ID NO: 4, light chain variable region CDR2 with a sequence of WAS, and light chain variable region CDR3 with a sequence as set forth in SEQ ID NO: 5.

In some embodiments of the present disclosure, the antibody or antigen binding fragment includes: (a) a heavy chain variable region with a sequence as set forth in SEQ ID NO: 6 and a light chain variable region with a sequence as set forth in SEQ ID NO: 7; or an amino acid sequence having at least 80% sequence identity to (a); (b) a heavy chain variable region with a sequence as set forth in SEQ ID NO: 8 and a light chain variable region with a sequence as set forth in SEQ ID NO: 9; or an amino acid sequence having at least 80% sequence identity to (b).

In some embodiments of the present disclosure, the antibody or antigen binding fragment includes: a heavy chain variable region with a sequence as set forth in SEQ ID NO: 6 or SEQ ID NO: 8; and/or a light chain variable region with a sequence as set forth in SEQ ID NO: 7 or SEQ ID NO:9.

In some embodiments of the present disclosure, the antibody or antigen binding fragment includes: a heavy chain variable region with a sequence as set forth in SEQ ID NO: 6 and a light chain variable region with a sequence as set forth in SEQ ID NO: 7; or a heavy chain variable region with a sequence as set forth in SEQ ID NO: 8 and a light chain variable region with a sequence as set forth in SEQ ID NO: 9.

In some embodiments of the present disclosure, the antibody or antigen binding fragment includes at least one of a heavy chain constant region and a light chain constant region, and at least a portion of the at least one of the heavy chain constant region and the light chain constant region is derived from at least one of a primate antibody and a murine antibody or a mutant thereof.

In some embodiments of the present disclosure, the light chain constant region and the heavy chain constant region are both derived from a murine IgG antibody or a mutant thereof or a human IgG antibody or a mutant thereof.

In some embodiments of the present disclosure, the light chain constant region and the heavy chain constant region are both derived from a murine IgG1 antibody or a mutant thereof or a human IgG1 antibody or a mutant thereof.

In some embodiments of the present disclosure, the N-terminus of the heavy chain constant region is linked to the C-terminus of the heavy chain variable region, and the N-terminus of the light chain constant region is linked to the C-terminus of the light chain variable region.

In some embodiments of the present disclosure, the antibody or antigen binding fragment has a heavy chain with an amino acid sequence as set forth in SEQ ID NO: 10 or SEQ ID NO: 12 and a light chain with an amino acid sequence as set forth in SEQ ID NO: 11 or SEQ ID NO: 13.

In some embodiments of the present disclosure, the antibody or antigen binding fragment has a heavy chain with an amino acid sequence as set forth in SEQ ID NO: 10 and a light chain with an amino acid sequence as set forth in SEQ ID NO: 11; or the antibody or antigen binding fragment has a heavy chain with an amino acid sequence as set forth in SEQ ID NO: 12 and a light chain with an amino acid sequence as set forth in SEQ ID NO: 13.

In some embodiments of the present disclosure, the antibody or antigen binding fragment includes a monoclonal antibody or a polyclonal antibody; the monoclonal antibody includes at least one of a full-length antibody, Fv, a single-chain antibody, Fab, a single-domain antibody, and a minimal recognition unit; and the antibody or antigen binding fragment binds to an amino acid sequence as set forth in SEQ ID NO: 14.

In a second aspect, the present disclosure provides a bispecific binding molecule, which includes a first binding region including the antibody or antigen binding fragment according to the first aspect; and a second binding region having a CD3 binding activity.

In some embodiments of the present disclosure, the bispecific binding molecule includes a symmetric bispecific binding molecule or an asymmetric bispecific binding molecule. The bispecific binding molecule is an asymmetric bispecific binding molecule.

In some embodiments of the present disclosure, the first binding region of the bispecific binding molecule includes peptide chain 1 including a heavy chain variable region with a sequence as set forth in SEQ ID NO: 6 or SEQ ID NO: 8; and peptide chain 2 including a light chain variable region with a sequence as set forth in SEQ ID NO: 7 or SEQ ID NO: 9.

In some embodiments of the present disclosure, the second binding region includes at least one of a full-length antibody, Fv, a single-chain antibody, Fab, a single-domain antibody, and a minimal recognition unit having a CD3 binding activity.

In some embodiments of the present disclosure, the second binding region includes an anti-CD3 single-chain antibody.

The CD3×PD-L1 bispecific antibody according to the present disclosure can bind more strongly to tumor cells and promote T cells to exert the anti-cancer function.

In some embodiments of the present disclosure, the anti-CD3 single-chain antibody includes: an anti-CD3 antibody heavy chain variable region including heavy chain variable region CDR1 with a sequence as set forth in SEQ ID NO: 15, heavy chain variable region CDR2 with a sequence as set forth in SEQ ID NO: 16, and heavy chain variable region CDR3 with a sequence as set forth in SEQ ID NO: 17; and an anti-CD3 antibody light chain variable region including light chain variable region CDR1 with a sequence as set forth in SEQ ID NO: 18, light chain variable region CDR2 with a sequence as set forth in GTN, and light chain variable region CDR3 with a sequence as set forth in SEQ ID NO: 19.

In some embodiments of the present disclosure, the anti-CD3 single-chain antibody includes: a heavy chain variable region with a sequence as set forth in SEQ ID NO: 20; and a light chain variable region with a sequence as set forth in SEQ ID NO: 21.

In some embodiments of the present disclosure, the anti-CD3 single-chain antibody further includes a linking peptide. The N-terminus of the linking peptide is linked to the C-terminus of the anti-CD3 antibody heavy chain variable region, and the C-terminus of the linking peptide is linked to the N-terminus of the anti-CD3 antibody light chain variable region; or the N-terminus of the linking peptide is linked to the C-terminus of the anti-CD3 antibody light chain variable region, and the C-terminus of the linking peptide is linked to the N-terminus of the anti-CD3 antibody heavy chain variable region.

In some embodiments of the present disclosure, the linking peptide has an amino acid sequence of (GGGGS)n, where n is an integer greater than or equal to 1, preferably 1, 2, 3, 4, 5, 6, 7, 8, 9, or 10.

In some embodiments of the present disclosure, the anti-CD3 single-chain antibody has an amino acid sequence as set forth in SEQ ID NO: 22.

In some embodiments of the present disclosure, the first binding region further includes at least one of a first heavy chain constant region and a light chain constant region, and at least a portion of the at least one of the first heavy chain constant region and the light chain constant region is derived from at least one of a human antibody, a primate antibody, a murine antibody, or a mutant thereof.

In some embodiments of the present disclosure, the first heavy chain constant region and the light chain constant region are both derived from a human IgG antibody or a mutant thereof.

In some embodiments of the present disclosure, the first heavy chain constant region and the light chain constant region are both derived from a human IgG1 antibody or a mutant thereof.

In some embodiments of the present disclosure, the N-terminus of the first heavy chain constant region is linked to the C-terminus of the heavy chain variable region, and the N-terminus of the light chain constant region is linked to the C-terminus of the light chain variable region.

In some embodiments of the present disclosure, the peptide chain 1 has an amino acid sequence as set forth in SEQ ID NO: 12, and the peptide chain 2 has an amino acid sequence as set forth in SEQ ID NO: 13.

In some embodiments of the present disclosure, the peptide chain 1 and the peptide chain 2 are linked via a disulfide bond.

In some embodiments of the present disclosure, the second binding region further includes a second heavy chain constant region, and at least a portion of the second heavy chain constant region is derived from at least one of a human antibody, a primate antibody, a murine antibody, or a mutant thereof.

In some embodiments of the present disclosure, the second heavy chain constant region is derived from a human IgG antibody or a mutant thereof.

In some embodiments of the present disclosure, the second heavy chain constant region is derived from a human IgG1 antibody or a mutant thereof.

In some embodiments of the present disclosure, the N-terminus of the second heavy chain constant region is linked to the C-terminus of the anti-CD3 single-chain antibody.

In some embodiments of the present disclosure, the first heavy chain constant region and the second heavy chain constant region are linked via a knob-into-hole structure.

In a third aspect, the present disclosure provides an isolated polynucleotide, which encodes the antibody or antigen binding fragment according to the first aspect or encodes the bispecific binding molecule according to the second aspect.

In a fourth aspect, the present disclosure provides an expression vector, which carries the polynucleotide according to the third aspect.

In a fifth aspect, the present disclosure provides a recombinant cell, which carries the polynucleotide according to the third aspect or the expression vector according to the fourth aspect, or expresses the antibody or antigen binding fragment according to the first aspect, or encodes the bispecific binding molecule according to the second aspect.

In some embodiments of the present disclosure, the recombinant cell is obtained by introducing the expression vector according to the fourth aspect into a host cell.

In some embodiments of the present disclosure, the recombinant cell is a eukaryotic cell.

In some embodiments of the present disclosure, the recombinant cell is a mammalian cell.

In a sixth aspect, the present disclosure provides a composition, which includes the antibody or antigen binding fragment according to the first aspect, the bispecific binding molecule according to the second aspect, the polynucleotide according to the third aspect, the expression vector according to the fourth aspect, or the recombinant cell according to the fifth aspect.

In a seventh aspect, the present disclosure provides a method for preparing the antibody or antigen binding fragment according to the first aspect or the bispecific binding molecule according to the second aspect, which includes culturing the recombinant cell according to the fifth aspect.

In an eighth aspect, the present disclosure provides a medicament, which includes the antibody or antigen binding fragment according to the first aspect, the bispecific binding molecule according to the second aspect, the polynucleotide according to the third aspect, the expression vector according to the fourth aspect, the recombinant cell according to the fifth aspect, or the composition according to the sixth aspect.

In a ninth aspect, the present disclosure provides a kit, which includes the antibody or antigen binding fragment according to the first aspect, the bispecific binding molecule according to the second aspect, the polynucleotide according to the third aspect, the expression vector according to the fourth aspect, or the recombinant cell according to the fifth aspect.

In a tenth aspect, the present disclosure provides use of the antibody or antigen binding fragment according to first aspect, the bispecific antibody according to second aspect, the polynucleotide according to third aspect, the expression vector according to fourth aspect, the recombinant cell according to fifth aspect, or the composition according to sixth aspect in the manufacture of a medicament for preventing and/or treating a PD-L1-mediated disease including cancer.

In some embodiments of the present disclosure, the cancer includes at least one selected from: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

In an eleventh aspect, the present disclosure provides use of the bispecific binding molecule according to second aspect, the polynucleotide according to third aspect, the expression vector according to fourth aspect, the recombinant cell according to fifth aspect, or the composition according to sixth aspect in the manufacture of a medicament for preventing and/or treating a PD-L1- and CD3-mediated disease.

In some embodiments of the present disclosure, the PD-L1- and CD3-mediated disease includes cancer.

In some embodiments of the present disclosure, the cancer includes at least one of: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

In a twelfth aspect, the present disclosure provides use of the antibody or antigen binding fragment according to first aspect, the bispecific antibody according to second aspect, the polynucleotide according to third aspect, the expression vector according to fourth aspect, or the recombinant cell according to fifth aspect in the manufacture of a kit for detecting PD-L1.

In a thirteenth aspect, the present disclosure provides use of the bispecific binding molecule according to second aspect, the polynucleotide according to third aspect, the expression vector according to fourth aspect, or the recombinant cell according to fifth aspect in the manufacture of a kit for detecting PD-L1 and/or CD3.

By utilizing an antibody-fusion protein composed of the CD3 antibody according to the present disclosure and the extracellular segment protein of the PD-1 receptor to bridge T cells with PD-L1-positive tumor cells, the ability of T cells to kill tumors can be promoted. Even if tumor cells express low levels of PD-L1, T cells can still promote complete tumor killing.

In a fourteenth aspect, the present disclosure provides use of the antibody or antigen binding fragment according to the first aspect, the bispecific antibody according to the second aspect, the polynucleotide according to the third aspect, the expression vector according to the fourth aspect, the recombinant cell according to the fifth aspect, the composition according to the sixth aspect, or the medicament according to the eighth aspect in the prevention and/or treatment of a disease. The disease includes cancer, and the cancer is characterized by positive PD-L1 on the surface of cancer cells.

According to a specific embodiment of the present disclosure, the cancer is selected from at least one of: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

In a fifteenth aspect, the present disclosure provides a method for treating a tumor, which includes administering to a subject at least one of: the antibody or antigen binding fragment according to first aspect, the bispecific antibody according to second aspect, the polynucleotide according to third aspect, the expression vector according to fourth aspect, the recombinant cell according to fifth aspect, the composition according to sixth aspect, or the medicament according to eighth aspect.

According to a specific embodiment of the present disclosure, the tumor is characterized by tumor cells that are positive for surface expression of PD-L1, and the tumor is selected from at least one of: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

Additional aspects and advantages of the present disclosure will be provided at least in part in the following description, or will become apparent at least in part from the following description, or can be learned from practicing of the present disclosure.

### BRIEF DESCRIPTION OF THE DRAWINGS

The above and/or additional aspects and advantages of the present disclosure will become apparent and easily understandable from the following description of embodiments in conjunction with the accompanying drawings, in which:
FIG. 1 is a graph showing the ELISA results of a murine 19B8 antibody binding to human (left panel A) and cynomolgus monkey PD-L1 (right panel B) proteins according to one embodiment of the present disclosure;
FIG. 2 is a graph showing the ELISA results of a murine 19B8 antibody blocking the binding of PD-1 to PD-L1 according to one embodiment of the present disclosure;
FIG. 3 is a graph showing the flow cytometry results of a murine 19B8 antibody binding to human lung cancer A549 cells according to one embodiment of the present disclosure;
FIG. 4 is a graph showing the ELISA results of a 19B8 chimeric antibody binding to human (left panel A) and cynomolgus monkey PD-L1 (right panel B) proteins according to one embodiment of the present disclosure;
FIG. 5 is a graph showing the ELISA results of a 19B8 chimeric antibody blocking the binding of PD-1 to PD-L1 according to one embodiment of the present disclosure;
FIG. 6 is a graph showing the flow cytometry results of a 19B8 chimeric antibody binding to human melanoma A375 cells (left panel A) and human lung cancer A549 cells (right panel B) according to one embodiment of the present disclosure;
FIG. 7 is a graph showing the ELISA results of a humanized 19B8 antibody blocking the binding of PD-1 to PD-L1 according to one embodiment of the present disclosure;
FIG. 8 is a graph showing the flow cytometry results of the binding of a humanized 19B8 antibody to human melanoma A375 cells (left panel A) and human lung cancer A549 cells (right panel B) according to one embodiment of the present disclosure;
FIG. 9 is a graph showing the results of a humanized 19B8 antibody promoting SEB-induced IL-2 secretion by human PBMCs according to one embodiment of the present disclosure;
FIG. 10 is a schematic diagram of a CD3×PD-L1 bispecific antibody according to one embodiment of the present disclosure;
FIG. 11 is a graph showing the results of a CD3×PD-L1 bispecific antibody binding to PD-L1 fusion protein according to one embodiment of the present disclosure;
FIG. 12 is a graph showing the ELISA results of a CD3×PD-L1 bispecific antibody blocking the binding of PD-1 to PD-L1 according to one embodiment of the present disclosure;
FIG. 13 is a graph showing the results of a CD3×PD-L1 bispecific antibody promoting PBMC to kill human melanoma A375 cells according to one embodiment of the present disclosure;
FIG. 14 is a graph showing the flow cytometry result of a CD3×PD-L1 bispecific antibody binding to human lung adenocarcinoma A549 cells according to one embodiment of the present disclosure;
FIG. 15 is a graph showing the results of a CD3×PD-L1 bispecific antibody promoting PBMC to kill human melanoma A375 cells according to one embodiment of the present disclosure; and
FIG. 16 is a graph showing the results of a CD3×PD-L1 antibody promoting anti-cancer effects in PBMC-reconstructed mice according to one embodiment of the present disclosure.

### DETAILED DESCRIPTION

Embodiments of the present disclosure are described in detail below. The embodiments described below are illustrative, and are merely used for explaining the present disclosure, and cannot be understood as a limitation to the present disclosure.

In addition, terms "first" and "second" are only used for descriptive purposes, and cannot be understood as indicating or implying relative importance, or implicitly indicating the number of indicated technical features. Therefore, the features defined with "first" and "second" may explicitly or implicitly include at least one of the features. In the description of the present disclosure, "plurality/more" means at least two, such as two or three, unless otherwise defined.

The endpoint values or any values of the ranges disclosed herein are not limited to the precise ranges or values. These ranges or values should be understood to include values approximating such ranges or values. For numerical ranges, one or more new numerical ranges can be obtained by combining the endpoint values of the respective ranges, an endpoint value and an individual point value within the respective ranges, and individual point values within the respective ranges with each other, and these numerical ranges should be regarded as specifically disclosed herein.

For an easier understanding of the present disclosure, certain technical and scientific terms are specifically defined below. Unless obviously and clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

As used herein, the terms "include" or "comprise" are open expressions, that is, including the contents specified in the present disclosure but not excluding other contents.

As used herein, the terms "optionally" or "optional" generally mean that the subsequently described event or circumstance may but need not occur, and that the description includes instances where the event or circumstance occurs and instances where it does not.

The antibody or antigen binding fragment of the present disclosure is typically prepared by biological synthesis methods. Based on the nucleotide sequences of the present disclosure, those skilled in the art can conveniently prepare an encoding nucleic acid of the present disclosure through various known methods. These methods include, but are not limited to, PCR, DNA synthesis, etc. Specific methods can be found in Molecular Cloning: A Laboratory Manual written by J. Sambrook. As an embodiment of the present disclosure, encoding nucleic acid sequences of the present disclosure can be constructed by synthesizing nucleotide sequences by stages and performing overlap extension PCR. The antibody or antigen fragment is numbered and defined using the Kabat numbering system. As used herein, the term "antibody" is an immunoglobulin molecule capable of binding to a specific antigen. It includes two light chains with lighter molecular weight and two heavy chains with heavier molecular weight. The heavy chain (H chain) and the light chain (L chain) are linked by disulfide bonds to form a tetrapeptide chain molecule. The amino acid sequence at the amino terminus (N terminus) of the peptide chain varies greatly, which is called the variable region (V region), while the carboxyl terminus (C terminus) is relatively stable and varies very little, which is called the constant region (C region). The V regions of the L chain and H chain are called VL and VH, respectively. In the variable region, the amino acid composition and arrangement order of some regions have a higher degree of variation, which is called the hypervariable region (HVR). The hypervariable region is the location where the antigen and antibody bind, so it is also called the complementarity-determining region (CDR). There are three CDR regions on both the heavy chain variable region and the light chain variable region.

The antibody of the present disclosure includes a murine antibody, a chimeric antibody, and a humanized antibody, preferably a humanized antibody.

As used herein, the term "murine antibody" refers to a monoclonal antibody against human B7H6 prepared according to the knowledge and skills in the art. The preparation is accomplished by injecting a test subject with the antigen and then isolating hybridomas that express antibodies having the desired sequence or functional characteristics. In one preferred embodiment of the present disclosure, the murine B7H6 antibody or the antigen binding fragment thereof may further include a light chain constant region of murine κ chain, λ chain, or variants thereof, or further include a heavy chain constant region of murine IgG1, IgG2, IgG3, or variants thereof.

In the present disclosure, the term "antigen binding fragment" or "antibody fragment" generally refers to an antigen-binding antibody fragment and may include a portion of an intact antibody, typically an antigen binding region or variable region. Examples of antibody fragments include Fab, Fab', F(ab')2, Fv or scFv, diabody, linear antibody, single-chain antibody molecules, etc.

In the present disclosure, the term "monoclonal antibody" or "mAb" refers to an antibody having a single antigen-binding site.

In the present disclosure, the term "bispecific antibody" or "bsAb" refers to an antibody having two different antigen-binding sites.

In the present disclosure, the term "mutant" or "variant" may refer to molecules obtained by performing mutation of one or more nucleotides or amino acids on any natural or engineered molecule.

In the present disclosure, the term "complementarity determining region" or "CDR" or "CDR sequence" refers to the amino acid sequence responsible for antigen binding in the antibody, for example, generally includes amino acid residues near 23-34 (L1), 50-56 (L2), and 89-97 (L3) in the light chain variable region, and amino acid residues near 31-35B (H1), 50-65 (H2), and 95-102 (H3) in the heavy chain variable region (Kabat et al., Sequences of Proteins of Immunological Interest, 5th Ed. Public Health Service, National Institutes of Health, Bethesda, MD.(1991)); and/or amino acid residues from the "hypervariable loops" (e.g., amino acid residues near 26-32 (LI), 50-52 (L2), and 91-96 (L3) in the light chain variable region, and 26-32 (H1), 53-55 (H2), and 96-101 (H3) in the heavy chain variable region (Chothia and Lesk J.Mol.Biol.196: 901-917(1987)).

In the present disclosure, the term "functional fragment" refers in particular to a fragment of an antibody such as Fv, scFv (sc means single chain), Fab, F(ab')2, Fab', scFv-Fc fragment or diabody, or any fragment which is supposed to be capable of increasing the half-life by chemical modification, e.g. addition of polyalkylene glycols, such as polyethylene glycol ("PEGylation, PEG") (referred to as PEGylated fragments of Fv-PEG, scFv-PEG, Fab-PEG, F(ab')2-PEG or Fab'-PEG) ("PEG" means polyethylene glycol), or by incorporation into liposomes.

In the present disclosure, the term "chimeric antibody" refers to a recombinant antibody obtained by replacing the amino acid sequence of the constant region of a monoclonal antibody from one species (e.g. mouse) with the constant region of an antibody from another species (e.g. human) using recombinant DNA technology.

In the present disclosure, the term "humanized antibody" refers to a recombinant antibody obtained by completely replacing the constant region and the non-CDR of variable region (Fv framework region (FR)) of a monoclonal antibody from one species (e.g. mouse) with the constant region and the non-CDR of variable region of an antibody from another species (e.g. human) using recombinant DNA technology. That is, when the constant region of an antibody is humanized, it is referred to as a chimeric antibody, while when the constant region and the non-CDR of variable region are both humanized, it is referred to as a humanized antibody.

In the present disclosure, a "full-length antibody" has a tetrapeptide chain structure formed by linking two identical light chains and two identical heavy chains via interchain disulfide bonds, such as immunoglobulin G (IgG), immunoglobulin A (IgA), immunoglobulin M (IgM), immunoglobulin D (IgD), or immunoglobulin E (IgE). The same class of immunoglobulins can also be divided into different subclasses based on their amino acid composition, such as IgG1, IgG2, IgG3, and IgG4. Immunoglobulin light chains are classified as either κ chains or λ chains depending on the constant region.

In the present disclosure, a "knob-into-hole structure" refers to a knob-hole mutation formed in the CH3 region of the heavy chain constant region of the antibody to facilitate a heavy chain engagement and form a heterodimer.

As used herein, when the term "identity" is used for describing amino acid sequences or nucleic acid sequences relative to reference sequences, a percentage of the same amino acid or nucleotide between two amino acid sequences or nucleic acid sequences is determined by conventional methods, for example, see Ausubel et al., eds. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl.3 (National Biomedical Research Foundation, Washington, D.C.). There are many algorithms for aligning sequences and determining sequence identity, including the homology alignment algorithm of Needleman et al., (1970) J.Mol.Biol.48:443; the local homology algorithm of Smith et al., (1981) Adv.Appl.Math.2:482; the similarity search method of Pearson et al., (1988) Proc.Natl.Acad.Sci.85:2444; the Smith-Waterman algorithm (Meth.Mol.Biol.70:173-187(1997)); and the BLASTP, BLASTN, and BLASTX algorithms (see Altschul et al., (1990) J.Mol.Biol.215:403-410). Computer programs utilizing these algorithms are also available, including but not limited to: ALIGN or Megalign (DNASTAR) software, or WU-BLAST-2 (Altschul et al., Meth.Enzym., 266:460-480(1996)); or GAP, BESTFIT, BLAST, Altschul *et al.,* ibid, FASTA, and TFASTA available in the Genetics Computing Group (GCG) package, Version 8, Madison, Wisconsin, USA; and CLUSTAL in the PC/Gene program provided by Intelligenetics, Mountain View, California.

Without substantially affecting antibody activity (remaining at least 95% of activity), substitution, addition and/or deletion of one or more amino acids (such as, 1, 2, 3, 4, 5, 6, 7, 8, 9, or 10 or more) may be conducted by those skilled in the art on the sequences in the present disclosure, to obtain variants of sequences of the antibody or functional fragments thereof. These variants are considered to be included within the scope of the present disclosure. For example, amino acids having similar properties may be substituted in the variable region. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred to herein are shown in a manner from the N-terminus to the C-terminus.

### Anti-PD-L1 antibody or antigen binding fragment

According to a specific embodiment of the present disclosure, the present disclosure provides an antibody (anti-PD-L1 antibody) or antigen binding fragment that can specifically recognize PD-L1. The antibody or antigen binding fragment includes a CDR selected from at least one of the following sequences or amino acid sequences having at least 80% identity thereto: heavy chain variable region CDR sequences: SEQ ID NO: 1, SEQ ID NO: 2, or SEQ ID NO: 3; or light chain variable region CDR sequences: SEQ ID NO: 4, WAS, or SEQ ID NO: 5.

"WAS" in the CDR sequence refers to an amino acid sequence.

According to a specific embodiment of the present disclosure, the anti-PD-L1 antibody or antigen binding fragment includes: heavy chain variable region CDR1 with a sequence as set forth in SEQ ID NO: 1, heavy chain variable region CDR2 with a sequence as set forth in SEQ ID NO: 2, heavy chain variable region CDR3 with a sequence as set forth in SEQ ID NO: 3, light chain variable region CDR1 with a sequence as set forth in SEQ ID NO: 4, light chain variable region CDR2 with a sequence of WAS, and light chain variable region CDR3 with a sequence as set forth in SEQ ID NO: 5.

According to a specific embodiment of the present disclosure, the anti-PD-L1 antibody or antigen binding fragment includes: a heavy chain variable region with a sequence as set forth in SEQ ID NO: 6 or SEQ ID NO: 8; and/or a light chain variable region with a sequence as set forth in SEQ ID NO: 7 or SEQ ID NO:9.

According to a specific embodiment of the present disclosure, the anti-PD-L1 antibody or antigen binding fragment includes: a heavy chain variable region with a sequence as set forth in SEQ ID NO: 6 and a light chain variable region with a sequence as set forth in SEQ ID NO: 7; or a heavy chain variable region with a sequence as set forth in SEQ ID NO: 8 and a light chain variable region with a sequence as set forth in SEQ ID NO: 9.

According to the specific embodiments of the present disclosure, without substantially affecting antibody activity (remaining at least 95% of activity), substitution, addition and/or deletion of one or more amino acids (such as, 1, 2, 3, 4, 5, 6, 7, 8, 9, or 10 or more) may be conducted by those skilled in the art on the sequences in the present disclosure, to obtain variants of sequences of the antibody or functional fragments thereof.. These variants are considered to be included within the scope of the present disclosure. For example, amino acids having similar properties may be substituted in the variable region. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred to herein are shown in a manner from the N-terminus to the C-terminus. Those skilled in the art should be aware that the CDR sequences analyzed from different databases may be different, but these changes should be included in the protection scope of the present disclosure.

According to the specific embodiments of the present disclosure, the antibody of the present disclosure may be full length (e.g. IgG1 or IgG4 antibodies) or may include only an antigen binding portion (e.g. Fab, F(ab')2 or scFv fragment), or may be modified to affect function. The present disclosure includes anti-PD-L1 antibodies having modified glycosylation patterns. In some uses, it may be useful to make modifications to remove undesired glycosylation sites, or to eliminate the presence of fucose moieties on the oligosaccharide chains, e.g. to enhance antibody-dependent cell-mediated cytotoxicity (ADCC) function. In other uses, galactosylation modifications can be made to alter complement dependent cytotoxicity (CDC). After a series of modifications, the fragments of the present disclosure still have PD-L1 binding activity, in particular an amino acid sequence as set forth in SEQ ID NO: 10. Preferably, the functional fragment will consist of or contain partial sequences of the heavy chain variable region or light chain variable region of its source antibody, sufficient to retain the same binding specificity and sufficient affinity as its source antibody. For PD-L1, it is preferred to be at least 1/100 of its source antibody affinity, and in a more preferred manner, at least 1/10. Such function fragments will include a minimum of 5 amino acids, preferably 10, 15, 25, 50, and 100 contiguous amino acids of the antibody sequence from which they are derived.

According to the specific embodiments of the present disclosure, in order to further improve the biological acceptability of the antibody, a humanization treatment is performed on the antibody in the present disclosure. Methods of humanization may be carried out with reference to conventional antibody engineering techniques. The humanized antibody according to the present disclosure has a heavy chain variable region with a sequence as set forth in SEQ ID NO: 8 and a light chain variable region with a sequence as set forth in SEQ ID NO: 9.

### Nucleic acid molecule, recombinant vector, recombinant cell, and immunoconjugate

In the process of preparing or obtaining these antibodies, nucleic acid molecules expressing these antibodies can be connected to different vectors and then expressed in different cells to obtain the corresponding antibodies.

Thus, the present disclosure also provides an isolated nucleic acid encoding the antibody or antigen binding fragment thereof described above, as well as a recombinant vector and a transformant containing the nucleic acid. The nucleic acid molecule encodes the above antibody or antigen binding fragment. The nucleic acid is preferably an expression cassette obtained by genetic engineering means.

The recombinant vector may refer to either a cloning vector or an expression vector, and can be obtained by operably linking the nucleic acid to a commercially available vector (e.g. a plasmid or viral vector). Common plasmids include pSeTag2, PEE14, pMH3, and the like.

In some preferred embodiments, the nucleic acid molecule is species-optimized and is more easily expressed in mammalian cells.

The present disclosure also provides an expression vector, which includes the above-mentioned isolated nucleic acid molecule. When the above-mentioned isolated polynucleotide is linked to the vector, the polynucleotide can be directly or indirectly linked to the control elements on the vector, as long as these control elements can control translation, expression, or the like of the polynucleotide. Of course, these control elements may be directly derived from the vector itself or be exogenous, i.e., not derived from the vector itself. Of course, the polynucleotide can be operably linked to the control element. The "operably linked" herein means an exogenous gene is linked to a vector such that control elements in the vector, such as a transcriptional control sequence and a translational control sequence, can exert their intended function of regulating transcription and translation of the exogenous gene. Of course, the polynucleotide encoding the antibody heavy chain and light chain can be respectively and independently inserted into different vectors, and is commonly inserted into the same vector. For example, common vectors may be plasmids, bacteriophages, etc.

The present disclosure also provides a recombinant cell, which contains the expression vector. The expression vector can be introduced into mammalian cells to construct recombinant cells, which are then used to express the antibody or antigen binding fragment according to the present disclosure. By culturing the recombinant cells, the corresponding antibodies can be obtained. The host cell of the present disclosure may be a prokaryotic host cell, a eukaryotic host cell, or a bacteriophage. The prokaryotic host cell may be *E. coli, Bacillus subtilis, Streptomyces* or *Proteus mirabilis,* etc. The eukaryotic cells may be fungi such as *Pichia pastoris, Saccharomyces cerevisiae, Schizosaccharomyces pombe, Trichoderma,* insect cells such as *Mythimna separata,* plant cells such as tobacco, mammalian cells such as BHK cells, CHO cells, COS cells, myeloma cells, and the like. In some embodiments, the host cells of the present disclosure are preferably mammalian cells, more preferably BHK cells, CHO cells, NSO cells, or COS cells.

The immunoconjugates according to the present disclosure contain a therapeutic agent and the above-mentioned antibody or antigen binding fragment coupled to the therapeutic agent. The antibody or antigen binding fragment can be coupled to the therapeutic agent in a conventional manner.

The composition according to the present disclosure contains the above-mentioned antibody or antigen binding fragment and/or the above-mentioned immunoconjugate, and a pharmaceutically acceptable carrier. In certain embodiments, the composition includes combinations separated in time and/or space, as long as they can work together to achieve the purpose of the present disclosure. For example, the components contained in the composition may be administered to the subject as a whole, or may be administered to the subject separately. When the components contained in the composition are administered to the subject separately, the respective components may be administered to the subject simultaneously or sequentially.

### Medicament, kit, use in the manufacture of the medicament, and use in the manufacture of the kit

The present disclosure also provides a medicament, which includes the above-mentioned antibody or antigen binding fragment and a pharmaceutically acceptable carrier, and may also include the above-mentioned immunoconjugate, nucleic acid molecule, expression vector, or recombinant cell.

In some embodiments, these pharmaceutical compositions further include a pharmaceutically acceptable carrier, including any solvent, solid excipient, diluent, binder, disintegrant, or other liquid excipient, dispersant, flavoring agent or suspending agent, surfactant, isotonic agent, thickener, emulsifier, preservative, solid binder, glidant or lubricant, etc., suitable for a specific target dosage form. Except to the extent that any conventional excipients are incompatible with the compounds of the present disclosure, for example by producing any undesirable biological effect or interacting in a deleterious manner with any other component of the pharmaceutically acceptable composition, their use is contemplated by the present disclosure.

The composition of the present disclosure may also be administered in combination with each other, or combination with one or more other therapeutic compounds, for example, in combination with a chemotherapeutic agent. Thus, the composition may also contain a chemotherapeutic agent. The antibody or antibody binding fragment thereof, or immunoconjugate of the present disclosure may also be combined with a second therapeutic agent, exemplary agents of which include, but are not limited to, other agents that inhibit PD-L1 activity (including other antibodies or antigen binding fragments thereof, peptide inhibitors, small molecule antagonists, etc.) and/or agents that interfere with PD-L1 upstream or downstream signal transduction.

Typically, the antibody or antigen binding fragment thereof is administered in an effective amount, i.e. an amount sufficient to achieve the desired therapeutic and/or prophylactic effect, e.g. an amount that results in the prevention or amelioration of symptoms associated with the disease being treated, e.g. a disease associated with PD-L1. The effective amount of the composition administered to a subject will depend on the type and severity of the disease, as well as on the characteristics of the individual, such as general health, age, sex, weight, and drug tolerance; it will also depend on the severity and type of disease, and one skilled in the art will be able to determine appropriate dosages depending on such factors.

In some embodiments of the present disclosure, the kit for detecting PD-L1 in a sample according to the present disclosure contains the above-mentioned antibody or antigen binding fragment, pharmaceutically acceptable carrier, immunoconjugate, nucleic acid molecule, expression vector, or recombinant cell. In some embodiments, the sample may be a tissue of a patient suffering from a PD-L1-mediated disease, in particular suffering from transplant rejection, autoimmune disease, infectious disease, or cancer, more preferably suffering from at least one of lung cancer, liver cancer, ovarian cancer, cervical cancer, skin cancer, bladder cancer, colon cancer, breast cancer, glioma, kidney cancer, gastric cancer, esophageal cancer, oral squamous cell cancer or head-and-neck cancer. The kit may also include reagents conventionally used to detect PD-L1, such as coating solutions and the like.

The present disclosure also provides use of the above-mentioned antibody or antigen binding fragment, pharmaceutically acceptable carrier, immunoconjugate, nucleic acid molecule, expression vector, or recombinant cell in the manufacture of a medicament for preventing and/or treating a PD-L1-mediated disease. Preferably, the PD-L1-mediated disease is transplant rejection, an autoimmune disease, an infectious disease, or a cancer. More preferably, the cancer is a cancer expressing PD-L1. Further preferably, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head-and-neck cancer. Further preferably, the infectious disease includes but is not limited to, HIV virus infection and/or hepatitis B virus infection.

The present disclosure also relates to a method for preventing and/or treating a PD-L1-mediated disease (described above). The method includes: administering to a patient an effective amount of at least one of the antibody or antigen binding fragment, immunoconjugate, or composition of the present disclosure. The mode of administration may be oral, nasal, intradermal, subcutaneous, intramuscular, intravenous, or intraperitoneal.

The nucleic acids encoding the heavy and/or light chains of the antibodies of the present disclosure are within the scope of the present disclosure. According to the amino acid sequences of the heavy and/or light chains, the corresponding nucleic acid sequences can be readily obtained by the person skilled in the art, as shown in Table 1.

**[Table 1]**

| SEQ ID NO: | Sequence | Description |
|---|---|---|
| 1 | GYSFTGYY | 19B8 HCDR1 |
| 2 | INPYNGAT | 19B8 HCDR2 |
| 3 | VPTGMGYFLMDY | 19B8 HCDR3 |
| 4 | QDVGSA | 19B8 LCDR1 |
| WAS | WAS | 19B8 LCDR2 |
| 5 | QQFTSYPT | 19B8 LCDR3 |
| 6 | EVQLQQSGPDLVKPGASVKISCKASGYSFTGYYMH WVKQSHVKSLEWIGRINPYNGATTYSQNFKDKASL TVDKSSSTAYMELHSLTSEDSAVYYCVPTGMGYFL MDYWGQGTSVTVSS | Murine 19B8 VH |
| 7 | DIVMTQSHKFMSASVGDRVSITCKASQDVGSAVVW YRQKPGQSPKLLIYWASTRHTGVPDRFTGSGSGTDF TLTISNVQSEDLADYFCQQFTSYPTFGSGTRLEIK | Murine 19B8 VL |
| 8 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSS | Humanized 19B8 VH |
| 9 | DIQLTQSPSFLSASVGDRVTITCKASQDVGSAVVWY QQKPGKAPKLLIYWASTRHTGVPSRFSGSGSGTEFT LTISSLQPEDFATYFCQQFTSYPTFGQGTKLEIK | Humanized 19B8 VL |
| 10 | EVQLQQSGPDLVKPGASVKISCKASGYSFTGYYMH WVKQSHVKSLEWIGRINPYNGATTYSQNFKDKASL TVDKSSSTAYMELHSLTSEDSAVYYCVPTGMGYFL MDYWGQGTSVTVSSAKTTPPSVYPLAPGSAAQTNS MVTLGCLVKGYFPEPVTVTWNSGSLSSGVHTFPAVL QSDLYTLSSSVTVPSSPRPSETVTCNVAHPASSTKVD KKIVPRDCGCKPCICTVPEVSSVFIFPPKPKDVLTITL TPKVTCVVVDISKDDPEVQFSWFVDDVEVHTAQTQ PREEQFNSTFRSVSELPIMHQDWLNGKEFKCRVNSA AFPAPIEKTISKTKGRPKAPQVYTIPPPKEQMAKDKV SLTCMITDFFPEDITVEWQWNGQPAENYKNTQPIMN TNGSYFVYSKLNVQKSNWEAGNTFTCSVLHEGLH NHHTEKSLSHSPGK | Murine heavy chain 19B8 VH-mIgG1 |
| 11 | DIVMTQSHKFMSASVGDRVSITCKASQDVGSAVVW YRQKPGQSPKLLIYWASTRHTGVPDRFTGSGSGTDF TLTISNVQSEDLADYFCQQFTSYPTFGSGTRLEIKRA DAAPTVSIFPPSSEQLTSGGASVVCFLNNFYPKDINV KWKIDGSERQNGVLNSWTDQDSKDSTYSMSSTLTL TKDEYERHNSYTCEATHKTSTSPIVKSFNRNEC | Murine light chain 19B8 VL-mK |
| 12 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK | Humanized 19B8 heavy chain-hIgG1LALA |
| 13 | DIQLTQSPSFLSASVGDRVTITCKASQDVGSAVVWY QQKPGKAPKLLIYWASTRHTGVPSRFSGSGSGTEFT LTISSLQPEDFATYFCQQFTSYPTFGQGTKLEIKRTVA APSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC | Humanized 19B8 light chain-hK |
| 14 | MQIPQAPWPVVWAVLQLGWRPGWFLDSPDRPWNP PTFSPALLVVTEGDNATFTCSFSNTSESFVLNWYRM SPSNQTDKLAAFPEDRSQPGQDCRFRVTQLPNGRDF HMSVVRARRNDSGTYLCGAISLAPKAQIKESLRAEL RVTERRAEVPTAHPSPSPRPAGQFQTLVVGVVGGLL GSLVLLVWVLAVICSRAARGTIGARRTGQPLKEDPS AVPVFSVDYGELDFQWREKTPEPPVPCVPEQTEYAT IVFPSGMGTSSPARRGSADGPRSAQPLRPEDGHCSW PL | PD-1 |
| 15 | GFTFNTYA | CD3 HCDR1 |
| 16 | IRSKYNNYAT | CD3 HCDR2 |
| 17 | VRHGNFGNSYVSWFAY | CD3 HCDR3 |
| 18 | TGAVTTSNY | CD3 LCDR1 |
| GTN | GTN | CD3 LCDR2 |
| 19 | ALWYSNLWV | CD3 LCDR3 |
| 20 | EVQLLESGGGLVQPGGSLKLSCAASGFTFNTYAMN WVRQAPGKGLEWVARIRSKYNNYATYYADSVKDR FTISRDDSKNTAYLQMNNLKTEDTAVYYCVRHGNF GNSYVSWFAYWGQGTLVTVSS | CD3 VH |
| 21 | ELVVTQEPSLTVSPGGTVTLTCRSSTGAVTTSNYAN WVQQKPGQAPRGLIGGTNKRAPGTPARFSGSLLGG KAALTLSGVQPEDEAEYYCALWYSNLWVFGGGTK LTVL | CD3 VL |
| 22 | EVQLLESGGGLVQPGGSLKLSCAASGFTFNTYAMN WVRQAPGKGLEWVARIRSKYNNYATYYADSVKDR FTISRDDSKNTAYLQMNNLKTEDTAVYYCVRHGNF GNSYVSWFAYWGQGTLVTVSSGGGGSGGGGSGGG GSELVVTQEPSLTVSPGGTVTLTCRSSTGAVTTSNYA NWVQQKPGQAPRGLIGGTNKRAPGTPARFSGSLLG GKAALTLSGVQPEDEAEYYCALWYSNLWVFGGGT KLTVL | Anti-CD3 single-chain antibody |
| 23 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVCTL PPSREEMTKNQVSLSCAVKGFYPSDIAVEWESNGQP ENNYKTTPPVLDSDGSFFLVSKLTVDKSRWQQGNV FSCSVMHEALHNHYTQKSLSLSPGK | h19B8-hIgG1LALAh oss |
| 24 | EVQLLESGGGLVQPGGSLKLSCAASGFTFNTYAMN WVRQAPGKGLEWVARIRSKYNNYATYYADSVKDR FTISRDDSKNTAYLQMNNLKTEDTAVYYCVRHGNF GNSYVSWFAYWGQGTLVTVSSGGGGSGGGGSGGG GSELVVTQEPSLTVSPGGTVTLTCRSSTGAVTTSNYA NWVQQKPGQAPRGLIGGTNKRAPGTPARFSGSLLG GKAALTLSGVQPEDEAEYYCALWYSNLWVFGGGT KLTVLGGGGSPKSSDKTHTCPPCPAPEAAGGPSVFL FPPKPKDTLMISRTPEVTCVVVDVSHEDPEVKFNW YVDGVEVHNAKTKPREEQYNSTYRVVSVLTVLHQ DWLNGKEYKCKVSNKALPAPIEKTISKAKGQPREP QVYTLPPCREEMTKNQVSLWCLVKGFYPSDIAVEW ESNGQPENNYKTTPPVLDSDGSFFLYSKLTVDKSRW QQGNVFSCSVMHEALHNHYTQKSLSLSPGK | CD3-hIgG1LALAk bss |
| 25 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPCREEMTKNQVSLWCLVKGFYPSDIAVEWESNGQ PENNYKTTPPVLDSDGSFFLYSKLTVDKSRWQQGN VFSCSVMHEALHNHYTQKSLSLSPGK | h19B8-hIgG1LALAk bss |
| 26 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPCREEMTKNQVSLWCLVKGFYPSDIAVEWESNGQ PENNYKTTPPVLDSDGSFFLYSKLTVDKSRWQQGN VFSCSVMHEALHNHYTQKSLSLSPGKGGGGSGGGG SEVQLLESGGGLVQPGGSLKLSCAASGFTFNTYAM NWVRQAPGKGLEWVARIRSKYNNYATYYADSVKD RFTISRDDSKNTAYLQMNNLKTEDTAVYYCVRHGN FGNSYVSWFAYWGQGTLVTVSSGGGGSGGGGSGG GGSELVVTQEPSLTVSPGGTVTLTCRSSTGAVTTSNY ANWVQQKPGQAPRGLIGGTNKRAPGTPARFSGSLL GGKAALTLSGVQPEDEAEYYCALWYSNLWVFGGG TKLTVL | h19B8-hIgG1LALAk bssCross3 |
| 27 | QVQLVQSGAEVKKPGASVKVSCKASGYSFTGYYM HWVRQAPGQGLEWIGRINPYNGATTYSQNFKDRAT MTVDTSISTAYMELSRLRSDDTAVYYCVPTGMGYF LMDYWGQGTLVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVCTL PPSREEMTKNQVSLSCAVKGFYPSDIAVEWESNGQP ENNYKTTPPVLDSDGSFFLVSKLTVDKSRWQQGNV FSCSVMHEALHNHYTQKSLSLSPGKGGGGSGGGGS EVQLLESGGGLVQPGGSLKLSCAASGFTFNTYAMN WVRQAPGKGLEWVARIRSKYNNYATYYADSVKDR FTISRDDSKNTAYLQMNNLKTEDTAVYYCVRHGNF GNSYVSWFAYWGQGTLVTVSSGGGGSGGGGSGGG GSELVVTQEPSLTVSPGGTVTLTCRSSTGAVTTSNYA NWVQQKPGQAPRGLIGGTNKRAPGTPARFSGSLLG GKAALTLSGVQPEDEAEYYCALWYSNLWVFGGGT KLTVL | h19B8-hIgG1LALAh ossCross3 |
| 28 | QVQLVQSGAEVKKPGSSVKVSCKTSGDTFSTYAISW VRQAPGQGLEWMGGIIPIFGKAHYAQKFQGRVTITA DESTSTAYMELSSLRSEDTAVYFCARKFHFVSGSPFG MDVWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK | 12A4-hIgG1LALA |
| 29 | QVQLVQSGAEVKKPGSSVKVSCKTSGDTFSTYAISW VRQAPGQGLEWMGGIIPIFGKAHYAQKFQGRVTITA DESTSTAYMELSSLRSEDTAVYFCARKFHFVSGSPFG MDVWGQGTTVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVCTL PPSREEMTKNQVSLSCAVKGFYPSDIAVEWESNGQP ENNYKTTPPVLDSDGSFFLVSKLTVDKSRWQQGNV FSCSVMHEALHNHYTQKSLSLSPGK | 12A4-hIgG1LALAh oss |
| 30 | EIVLTQSPATLSLSPGERATLSCRASQSVSSYLAWYQ QKPGQAPRLLIYDASNRATGIPARFSGSGSGTDFTLT ISSLEPEDFAVYYCQQRSNWPTFGQGTKVEIKRTVA APSVFIFPPSDEQLKSGTASVVCLLNNFYPREAKVQ WKVDNALQSGNSQESVTEQDSKDSTYSLSSTLTLSK ADYEKHKVYACEVTHQGLSSPVTKSFNRGEC | 12A4-hK |
| 31 | EVQLQQSGPDLVKPGASVKISCKASGYSFTGYYMH WVKQSHVKSLEWIGRINPYNGATTYSQNFKDKASL TVDKSSSTAYMELHSLTSEDSAVYYCVPTGMGYFL MDYWGQGTSVTVSSASTKGPSVFPLAPSSKSTSGG TAALGCLVKDYFPEPVTVSWNSGALTSGVHTFPAVL QSSGLYSLSSVVTVPSSSLGTQTYICNVNHKPSNTK VDKKVEPKSCDKTHTCPPCPAPEAAGGPSVFLFPPK PKDTLMISRTPEVTCVVVDVSHEDPEVKFNWYVDG VEVHNAKTKPREEQYNSTYRVVSVLTVLHQDWLN GKEYKCKVSNKALPAPIEKTISKAKGQPREPQVYTL PPSREEMTKNQVSLTCLVKGFYPSDIAVEWESNGQP ENNYKTTPPVLDSDGSFFLYSKLTVDKSRWQQGNV FSCSVMHEALHNHYTQKSLSLSPGK | Murine 19B8 heavy chain-hIgG1LALA chimeric antibody |
| 32 | DIVMTQSHKFMSASVGDRVSITCKASQDVGSAVVW YRQKPGQSPKLLIYWASTRHTGVPDRFTGSGSGTDF TLTISNVQSEDLADYFCQQFTSYPTFGSGTRLEIKRT VAAPSVFIFPPSDEQLKSGTASVVCLLNNFYPREAKV QWKVDNALQSGNSQESVTEQDSKDSTYSLSSTLTLS KADYEKHKVYACEVTHQGLSSPVTKSFNRGEC | Murine 19B8 light chain-hK chimeric antibody |

Hereinafter, the scheme of the present disclosure will be explained in conjunction with examples. Those skilled in the art will understand that the following examples are only used to illustrate the present disclosure and should not be considered as limiting the scope of the present disclosure. Where specific techniques or conditions are not specified in the examples, they are performed according to techniques or conditions described in the literature in the art or according to the product specification. The reagents or instruments used without specifying the manufacturer are all conventional products that can be obtained commercially.

### Example 1: Antibody Preparation

C57BL/6 mice (9-week-old, purchased from Shanghai SLAC, weighing about 20 g) were immunized with purified recombinant PD-L1 extracellular region Strep tag fusion protein (PD-L1-strep) as an antigen to generate murine monoclonal antibody against human PD-L1.

Mice were immunized three times with purified antigen and a complete Freund's adjuvant, and immune responses were detected after exsanguination via the tail vein. Sera were screened by ELISA and flow cytometry to identify mice with anti-human PD-L1 immunoglobulin. Splenocytes were taken out from mice with the highest anti-PD-L1 immunoglobulin and fused with murine myeloma SP2/0 cells (ATCC NO.: CRL-1581). The fused hybridoma cells were subjected to antibody screening to obtain murine mAbs.

The candidate hybridoma cells were cultured to a total number of 10⁶ cells. The cells were collected by centrifugation at 800 rpm for 10 min, and the total RNA was extracted with a Trizol kit (Invitrogen). The total RNA was used as a template to synthesize the cDNA library (Invitrogen) by reverse transcription, and cDNA was used as a template to amplify the corresponding nucleic acid sequence of the variable region in hybridoma cells by PCR. The primer sequences used in the PCR amplification were complementary to first framework region of the antibody variable region or signal peptide region and the constant region (Larrick, J.W., et al., (1990) Scand. J. Immunol., 32, 121-128 and Coloma, J.J. et al., (1991) BioTechniques, 11, 152-156). In a 50 µL reaction system, 2 µL of cDNA, 5 µL of 10 × PCR buffer, 2 µL of upstream and downstream primers (5 µmol), 2 µL of dNTP, 1 µL of Taq enzyme (Takara, Ex Taq), and 38 µL of H₂O were added respectively. A pre-denaturation was performed for 5 min at 95°C, and the procedure entered the temperature cycle for PCR amplification. The reaction conditions included: denaturation at 94°C for 30 s, annealing at 58°C for 45 s, extension at 72°C for 50 s, for 32 cycles, then extension at 72°C for 7 min. After sequencing the amplification products, the heavy and light chain variable region sequences of murine mAb were obtained.

The amino acid sequence of the heavy chain variable region is as set forth in SEQ ID NO: 6:

EVQLQQSGPDLVKPGASVKISCKASGYSFTGYYMHWVKQSHVKSLEWIG
RINPYNGATTYSQNFKDKASLTVDKSSSTAYMELHSLTSEDSAVYYCVPTGMGYFLM
DYWGQGTSVTVSS

The amino acid sequence of the light chain variable region is as set forth in SEQ ID NO: 7:

DIVMTQSHKFMSASVGDRVSITCKASQDVGSAVVWYRQKPGQSPKLLIYW
ASTRHTGVPDRFTGSGSGTDFTLTISNVQSEDLADYFCQQFTSYPTFGSGTRLEIK

### Example 2: ELISA Assay for PD-L1 Antibody Binding

The ELISA assay was used to detect the binding characteristics of the PD-L1 antibody. The PD-L1 extracellular region protein was coated into a 96-well plate. The signal intensity after antibody addition was used to determine the binding characteristic of the antibody to PD-L1.

Antibody production: The pcDNA3.4 vectors containing the heavy chain and light chain nucleotide sequences of the above antibody (synthesized by Nanjing GenScript, murine 19B8 heavy chain, also referred to as 19B8-mIgG1, with an amino acid sequence as set forth in SEQ ID NO: 10, and murine h19B8 light chain, also referred to as 19B8-mK, with an amino acid sequence as set forth in SEQ ID NO: 11) were transiently transfected into ExpiCHO-S cells (Gibco, Catalog No. A29127) to prepare the antibody.

EVQLQQSGPDLVKPGASVKISCKASGYSFTGYYMHWVKQSHVKSLEWIG
RINPYNGATTYSQNFKDKASLTVDKSSSTAYMELHSLTSEDSAVYYCVPTGMGYFLM
DYWGQGTSVTVSSAKTTPPSVYPLAPGSAAQTNSMVTLGCLVKGYFPEPVTVTWNS
GSLSSGVHTFPAVLQSDLYTLSSSVTVPSSPRPSETVTCNVAHPASSTKVDKKIVPRDC
GCKPCICTVPEVSSVFIFPPKPKDVLTITLTPKVTCVVVDISKDDPEVQFSWFVDDVEV
HTAQTQPREEQFNSTFRSVSELPIMHQDWLNGKEFKCRVNSAAFPAPIEKTISKTKGRP
KAPQVYTIPPPKEQMAKDKVSLTCMITDFFPEDITVEWQWNGQPAENYKNTQPIMNT
NGSYFVYSKLNVQKSNWEAGNTFTCSVLHEGLHNHHTEKSLSHSPGK (SEQ ID NO:
10)

DIVMTQSHKFMSASVGDRVSITCKASQDVGSAVVWYRQKPGQSPKLLIYW
ASTRHTGVPDRFTGSGSGTDFTLTISNVQSEDLADYFCQQFTSYPTFGSGTRLEIKRAD
AAPTVSIFPPSSEQLTSGGASVVCFLNNFYPKDINVKWKIDGSERQNGVLNSWTDQDS
KDSTYSMSSTLTLTKDEYERHNSYTCEATHKTSTSPIVKSFNRNEC (SEQ ID NO: 11)

One day before transfection, the cell density of the ExpiCHO-S cells was adjusted to (3 to 4)×10⁶ cells/mL and the cells were cultured overnight at 37°C, 8% CO₂, and 95 rpm with shaking. On the day of transfection, when the cells grew to 7×10⁶ to 1×10⁷ cells/mL and the viability was greater than 95%, the transfection was started. The freshly preheated ExpiCHO medium (Gibco, catalog number A2910002) was used to dilute the cells to 6×10⁶ cells/mL, and the above-mentioned pcDNA3.4 plasmids carrying heavy and light chains (the ratio of light and heavy chain plasmids was 1:1) and ExpiFectamine CHO transfection reagent (Gibco, catalog number A29129) were transfected into the ExpiCHO-S cells. The cells were cultured with shaking at 37°C, 8% CO₂, and 95 rpm. 18 to 22 h after transfection, ExpiFectamine CHO Enhancer and ExpiCHO Feed were mixed well and immediately added to the transfected cells, and the mixture was mixed well and cultured with shaking at 32°C, 5% CO₂, and 95 rpm. On day 5 after transfection, 8 mL of ExpiCHO Feed was supplemented to the cells, mixed well, and continued to culture. The changes in cell number and cell viability were observed daily, and the cells were harvested by centrifugation when the cell viability dropped below 80% or after 10 to 14 days of the culture. The expressed supernatant was filtered with a 0.45 µm filter membrane, and the antibody with Fc domain was captured from the expressed supernatant using a Mabselect prism Aprotein A affinity chromatography column (purchased from Suzhou Nanovitamin). After the chromatography column was equilibrated with a pH 7.2 phosphate buffer, the supernatant was passed through the affinity chromatography column, eluted with an elution buffer (100 mM citric acid, pH 2.7), and finally concentrated and replaced with a PBS buffer. The purified antibody was identified by SDS-PAGE to have a purity of more than 95%. The results showed that the above-mentioned antibody was finally obtained.

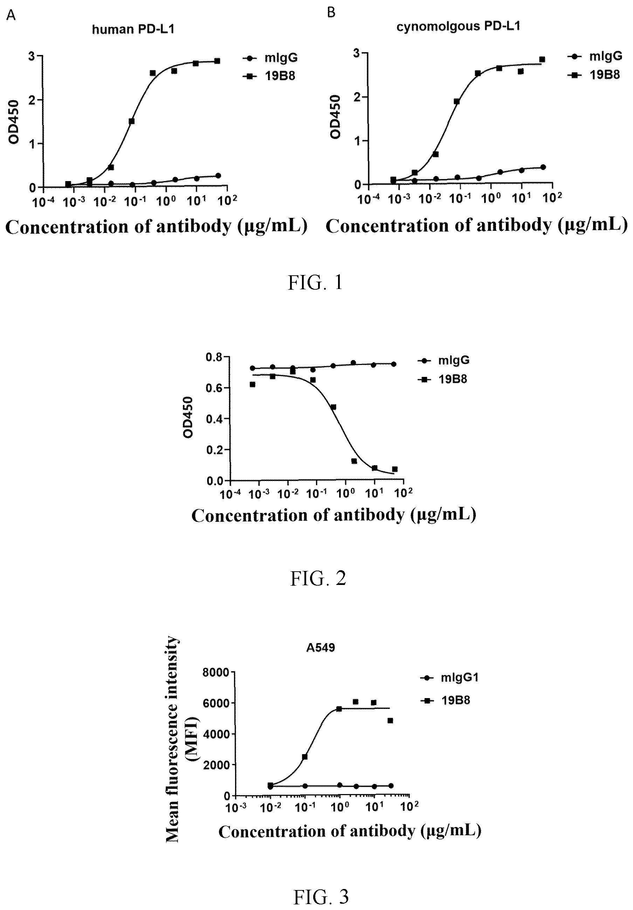

Human PD-L1-Fc fusion protein (purchased from Acro) or monkey PD-L1-His tag protein (purchased from Acro) was diluted to 1 µg/ml with PBS buffer, added to a 96-well plate at 100 µl/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, 100 µl/well of the murine PD-L1 antibody 19B8 to be tested diluted to an appropriate concentration with PBST/0.05% BSA was added and incubated at 37°C for 1 h. The reaction system was removed, and the plate was washed 6 times with PBST. HRP (horseradish peroxidase)-labeled anti-mouse IgG secondary antibody was diluted with PBST/0.05% BSA at 100 µl/well and incubated at 37°C for 1 h. After washing the plate 6 times with PBST, 80 µl/well TMB (tetramethylbenzidine) was added and incubated at room temperature for 3 min. 4 M sulfuric acid was added at 80 µl/well to terminate the reaction. The absorbance was read at 450 nm using a microplate reader. The results are shown in FIG. 1 and indicate that the PD-L1 antibody of the present disclosure can bind to both human and monkey PD-L1 proteins.

### Example 3: Detection Assay for Blocking Activity of Murine PD-L1 Antibody

The ELISA assay was used to detect the effect of the antibody on the binding of PD-L1 to its receptor PD-1. The specific assay was as follows.

PD-L1-Fc fusion protein (purchased from Acro) was diluted to 5 µg/mL with PBS buffer, added to a 96-well plate at 100 µL/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, 19B8 antibody diluted to different concentrations with PBST/0.05% BSA and PD-1-Avi tag (purchased from Acro) diluted to 2 µg/ml were added at 100 µL/well and incubated at 37°C for 1 h. The reaction system was removed, and the plate was washed 6 times with PBST. HRP (horseradish peroxidase)-labeled Strreptavidin secondary antibody (purchased from Southern biotech) was diluted with PBST/0.05% BSA at 100 µL/well and incubated at 37°C for 1 h. After washing the plate with PBST for 6 times, 80 µL/well TMB (tetramethylbenzidine) was added and incubated at room temperature for 3 min. 4 M sulfuric acid was added at 80 µL/well to terminate the reaction. mIgG (purchased from Biolegend) was used as a control. The absorbance was read at 450 nm using a microplate reader. The results are shown in FIG. 2 and indicate that the murine PD-L1 antibody (19B8) of the present disclosure can block the binding of PD-L1 to its receptor PD-1.

### Example 4: Flow Cytometry Assay for Murine PD-L1 Antibody Binding

Human lung adenocarcinoma A549 cells were diluted to 1×10⁶ cells/mL with PBS and added to a 1.5 mL EP tube at 100 µL/tube. 10 µL/tube of goat serum was added thereto and blocked at 4°C for 30 min. Different concentrations of 19B8 antibodies (heavy chain amino acid sequence is as set forth in SEQ ID NO: 10, and light chain amino acid sequence is as set forth in SEQ ID NO: 11) were added and incubated at 4°C for 30 min. 1 mL of PBS was added to the EP tube. The mixture was centrifuged at 4°C and 3500 rpm for 5 min. The supernatant was discarded, and the system was washed again with PBS. After centrifugation, the supernatant was discarded, and the cells were resuspended with 100 µL/tube PBS. 1 µL/tube of Alexa-647-labeled goat anti-mouse secondary antibody (Biolegend) was added and incubated at 4°C in the dark for 30 min. The system was washed twice with PBS, and the supernatant was discarded after centrifugation. The cells were resuspended with 200 µL/tube PBS and detected by flow cytometry. The results are shown in FIG. 3 and further demonstrate that the murine PD-L1 antibody of the present disclosure can bind to PD-L1 on the surface of tumor cells.

### Example 5: ELISA Assay for Human-Murine Chimeric PD-L1 Antibody Binding

The ELISA assay was used to detect the binding characteristics of the PD-L1 antibody. The PD-L1 extracellular region protein was coated into a 96-well plate. The signal intensity after antibody addition was used to determine the binding characteristic of the antibody to PD-L1.

Antibody production: The pcDNA3.4 vectors containing the heavy chain and light chain nucleotide sequences of the above antibody (synthesized by Nanjing GenScript, murine 19B8 heavy chain hIgG1LALA, also referred to as 19B8-hIgG1LALA, with an amino acid sequence as set forth in SEQ ID NO: 31, and murine h19B8 light chain hK, also referred to as 19B8-hK, with an amino acid sequence as set forth in SEQ ID NO: 32), fully human 12A4 antibody heavy chain 12A4-hIgG1LALA (amino acid sequence as set forth in SEQ ID NO: 28), and fully human 12A4 antibody light chain 12A4-hK (amino acid sequence as set forth in SEQ ID NO: 30) were transiently transfected into ExpiCHO-S cells (Gibco, Catalog No. A29127) to prepare the antibody.

EVQLQQSGPDLVKPGASVKISCKASGYSFTGYYMHWVKQSHVKSLEWIG
RINPYNGATTYSQNFKDKASLTVDKSSSTAYMELHSLTSEDSAVYYCVPTGMGYFLM

DYWGQGTSVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEPKSC
DKTHTCPPCPAPEAAGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIEK
TISKAKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPGK
(SEQ ID NO: 31)

DIVMTQSHKFMSASVGDRVSITCKASQDVGSAVVWYRQKPGQSPKLLIYW
ASTRHTGVPDRFTGSGSGTDFTLTISNVQSEDLADYFCQQFTSYPTFGSGTRLEIKRTV
AAPSVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC (SEQ ID NO: 32)

QVQLVQSGAEVKKPGSSVKVSCKTSGDTFSTYAISWVRQAPGQGLEWMG
GIIPIFGKAHYAQKFQGRVTITADESTSTAYMELSSLRSEDTAVYFCARKFHFVSGSPFG
MDVWGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 28)

EIVLTQSPATLSLSPGERATLSCRASQSVSSYLAWYQQKPGQAPRLLIYDAS
NRATGIPARFSGSGSGTDFTLTISSLEPEDFAVYYCQQRSNWPTFGQGTKVEIKRTVAAP
SVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 30)

One day before transfection, the cell density of the ExpiCHO-S cells was adjusted to (3 to 4)×10⁶ cells/mL and the cells were cultured overnight at 37°C, 8% CO₂, and 95 rpm with shaking. On the day of transfection, when the cells grew to 7×10⁶ to 1×10⁷ cells/mL and the viability was greater than 95%, the transfection was started. The freshly preheated ExpiCHO medium (Gibco, catalog number A2910002) was used to dilute the cells to 6×10⁶ cells/mL, and the above-mentioned pcDNA3.4 plasmid carrying heavy and light chains (the ratio of light and heavy chain plasmids was 1:1) and ExpiFectamine CHO transfection reagent (Gibco, catalog number A29129) were transfected into the ExpiCHO-S cells. The cells were cultured with shaking at 37°C, 8% CO₂, and 95 rpm. 18 to 22 h after transfection, ExpiFectamine CHO Enhancer and ExpiCHO Feed were mixed well and immediately added to the transfected cells, and the mixture was mixed well and cultured with shaking at 32°C, 5% CO₂, and 95 rpm. On day 5 after transfection, 8 mL of ExpiCHO Feed was supplemented to the cells, mixed well, and continued to culture. The changes in cell number and cell viability were observed daily, and the cells were harvested by centrifugation when the cell viability dropped below 80% or after 10 to 14 days of the culture. The expressed supernatant was filtered with a 0.45 µm filter membrane, and the antibody with Fc domain was captured from the expressed supernatant using a Mabselect prism Aprotein A affinity chromatography column (purchased from Suzhou Nanovitamin). After the chromatography column was equilibrated with a pH 7.2 phosphate buffer, the supernatant was passed through the affinity chromatography column, eluted with an elution buffer (100 mM citric acid, pH 2.7), and finally concentrated and replaced with a PBS buffer. The purified antibody was identified by SDS-PAGE to have a purity of more than 95%. The results showed that the above-mentioned antibody was finally obtained.

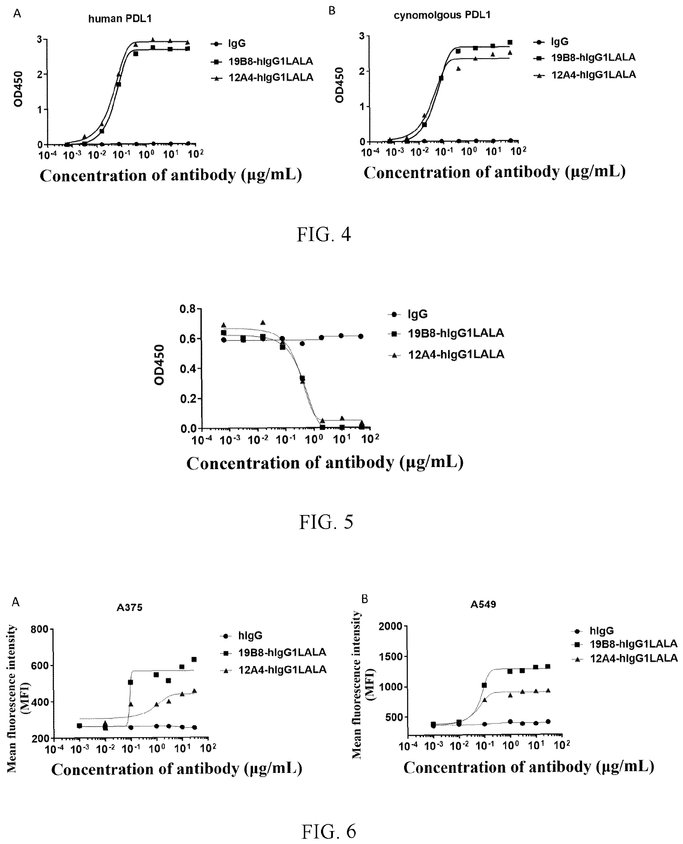

Human PD-L1-Fc fusion protein (purchased from Acro) or monkey PD-L1-His tag fusion protein (purchased from Acro) was diluted to 1 µg/ml with PBS buffer, added to a 96-well plate at 100 µl/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, the chimeric antibody 19B8-hIgGLALA to be tested diluted to an appropriate concentration with PBST/0.05% BSA and the control antibody 12A4-hIgG1LALA were added at 100 µl/well and incubated at 37°C for 1 h. The reaction system was removed, and the plate was washed 6 times with PBST. HRP (horseradish peroxidase)-labeled anti-human IgG-Fab secondary antibody was diluted with PBST/0.05% BSA at 100 µl/well and incubated at 37°C for 1 h. After washing the plate 6 times with PBST, 80 µl/well TMB (tetramethylbenzidine) was added and incubated at room temperature for 3 min. 4 M sulfuric acid was added at 80 µL/well to terminate the reaction. The absorbance was read at 450 nm using a microplate reader. The results are shown in FIG. 4 and indicate that the chimeric antibody 19B8-hIgG1LALA of the present disclosure can bind to both human and monkey PD-L1 proteins.

### Example 6: Detection Assay for Blocking Activity of Human-Murine Chimeric PD-L1 Antibody

The ELISA assay was used to detect the effect of the chimeric antibody on the binding of PD-L1 to its receptor PD-1. The specific assay was as follows.

PD-L1-Fc fusion protein (purchased from Acro) was diluted to 5 µg/mL with PBS buffer, added to a 96-well plate at 100 µL/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. 19B8-hIgG1LALA antibody or 12A4-hIgG1LALA antibody diluted to different concentrations with PBST/0.05% BSA and 2 µg/ml PD-1-Avi tag protein (purchased from Acro) were added at 100 µL/well, PBST/0.05% BSA was added at 100 µL/well, and the system was incubated at 37°C for 1 h. The reaction system was removed, and the plate was washed 6 times with PBST. HRP (horseradish peroxidase)-labeled Strreptavidin secondary antibody (purchased from Southern biotech) was diluted with PBST/0.05% BSA at 100 µL/well and incubated at 37°C for 1 h. After washing the plate with PBST for 6 times, 80 µL/well TMB (tetramethylbenzidine) was added and incubated at room temperature for 3 min. 4 M sulfuric acid was added at 80 µL/well to terminate the reaction. mIgG (purchased from Biolegend) was used as a control. The absorbance was read at 450 nm using a microplate reader. The results are shown in FIG. 5 and indicate that the chimeric antibody 19B8-hIgG1LALA of the present disclosure can block the binding of PD-L1 to its receptor PD-1.

### Example 7: Flow Cytometry Assay for Human-Murine Chimeric PD-L1 Antibody Binding

Human melanoma A375 cells and human lung adenocarcinoma A549 cells were diluted to 1×10⁶ cells/mL with PBS and added to 1.5 mL EP tubes at 100 µL/tube. 10 µL/tube of rat serum was added and blocked at 4°C for 30 min. Different concentrations of chimeric antibody 19B8-hIgG1LALA or control antibody 12A4-hIgG1LALA were added and incubated at 4°C for 30 min. 1 mL of PBS was added to the EP tube. The system was centrifuged at 4°C and 3500 rpm for 5 min. The supernatant was discarded, and the system was washed again with PBS. After centrifugation, the supernatant was discarded, and the cells were resuspended with 100 µL/tube PBS. 1 µL/tube of Alexa-647-labeled rat anti-human secondary antibody (Biolegend) was added and incubated at 4°C in the dark for 30 min. The system was washed twice with PBS, and the supernatant was discarded after centrifugation. The cells were resuspended with 200 µL/tube PBS and detected by flow cytometry. The results are shown in FIG.6 and further demonstrate that the chimeric antibody of the present disclosure can bind to the PD-L1 protein on the surface of tumor cells, and its binding is stronger than that of the control antibody 12A4.

### Example 8: Humanization of Mouse Antibody

Referring to the light chain variable region sequence and heavy chain variable region sequence of the PD-L1 antibody, a humanized template that best matched its non-CDR region was selected. The CDRs of the murine antibody was grafted onto the selected humanized template to replace the CDRs of the human template to obtain a humanized antibody. Then, based on the three-dimensional structure of the murine antibody, the buried residues, the residues that directly interact with the CDRs, and the residues that have an important influence on the conformation of VL and VH were back mutated to obtain the humanized antibody. The sequence of the heavy chain variable region of the humanized PD-L1 antibody is as set forth in SEQ ID NO: 8, and the sequence of the light chain variable region thereof is as set forth in SEQ ID NO: 9.

### Example 9: Detection Assay for Blocking Activity of Humanized PD-L1 Antibody

The ELISA assay was used to detect the effect of the antibody on the binding of PD-L1 to its receptor PD-1. The specific assay was as follows.

Antibody production: The pcDNA3.4 vectors containing the heavy chain and light chain nucleotide sequences of the above antibody (synthesized by Nanjing GenScript, humanized 19B8 heavy chain hIgG1LALA, also referred to as h19B8-hIgG1LALA, with an amino acid sequence as set forth in SEQ ID NO: 12, and humanized h19B8 light chain hK, also referred to as h19B8-hK, with an amino acid sequence as set forth in SEQ ID NO: 13) were transiently transfected into ExpiCHO-S cells (Gibco, Catalog No. A29127) to prepare the antibody.

QVQLVQSGAEVKKPGASVKVSCKASGYSFTGYYMHWVRQAPGQGLEWI
GRINPYNGATTYSQNFKDRATMTVDTSISTAYMELSRLRSDDTAVYYCVPTGMGYFL
MDYWGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSPGK
(SEQ ID NO: 12)

DIQLTQSPSFLSASVGDRVTITCKASQDVGSAVVWYQQKPGKAPKLLIYWA
STRHTGVPSRFSGSGSGTEFTLTISSLQPEDFATYFCQQFTSYPTFGQGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID NO: 13)

One day before transfection, the cell density of the ExpiCHO-S cells was adjusted to (3 to 4)×10⁶ cells/mL and the cells were cultured overnight at 37°C, 8% CO₂, and 95 rpm with shaking. On the day of transfection, when the cells grew to 7×10⁶ to 1×10⁷ cells/mL and the viability was greater than 95%, the transfection was started. The freshly preheated ExpiCHO medium (Gibco, catalog number A2910002) was used to dilute the cells to 6×10⁶ cells/mL, and the above-mentioned pcDNA3.4 plasmid carrying heavy and light chains (the ratio of light and heavy chain plasmids was 1:1) and ExpiFectamine CHO transfection reagent (Gibco, catalog number A29129) were transfected into the ExpiCHO-S cells. The cells were cultured with shaking at 37°C, 8% CO₂, and 95 rpm. 18 to 22 h after transfection, ExpiFectamine CHO Enhancer and ExpiCHO Feed were mixed well and immediately added to the transfected cells, and the mixture was mixed well and cultured with shaking at 32°C, 5% CO₂, and 95 rpm. On day 5 after transfection, 8 mL of ExpiCHO Feed was supplemented to the cells, mixed well, and continued to culture. The changes in cell number and cell viability were observed daily, and the cells were harvested by centrifugation when the cell viability dropped below 80% or after 10 to 14 days of the culture. The expressed supernatant was filtered with a 0.45 µm filter membrane, and the antibody with Fc domain was captured from the expressed supernatant using a Mabselect prism Aprotein A affinity chromatography column (purchased from Suzhou Nanovitamin). After the chromatography column was equilibrated with a pH 7.2 phosphate buffer, the supernatant was passed through the affinity chromatography column, eluted with an elution buffer (100 mM citric acid, pH 2.7), and finally concentrated and replaced with a PBS buffer. The purified antibody was identified by SDS-PAGE to have a purity of more than 95%. The results showed that the above-mentioned antibody was finally obtained.

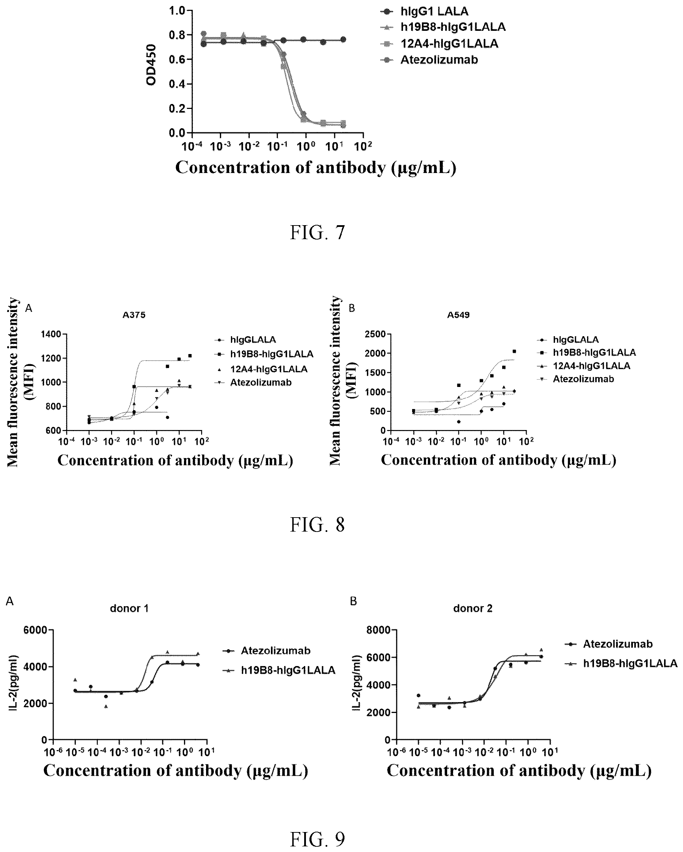

PD-L1-Fc fusion protein (purchased from Acro) was diluted to 5 µg/mL with PBS buffer, added to a 96-well plate at 100 µL/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, the humanized PD-L1 antibody h19B8-hIgG1LALA to be tested or control antibody 12A4-hIgG1LALA diluted to an appropriate concentration with PBST/0.05% BSA, Atezulizumab analog (purchased from Bio-Tech), and 2 µg/mL PD-1-Avi tag (purchased from Acro) were added at 100 µL/well and incubated at 37°C for 1 h. The reaction system was removed, and the plate was washed 6 times with PBST. HRP (horseradish peroxidase)-labeled Strreptavidin secondary antibody (purchased from Southern biotech) was diluted with PBST/0.05% BSA at 100 µL/well and incubated at 37°C for 1 h. After washing the plate with PBST for 6 times, 80 µL/well TMB (tetramethylbenzidine) was added and incubated at room temperature for 3 min. 4 M sulfuric acid was added at 80 µL/well to terminate the reaction. mIgG (purchased from Biolegend) was used as a control. The absorbance was read at 450 nm using a microplate reader. The results are shown in FIG. 7 and indicate that the humanized PD-L1 antibody h19B8 of the present disclosure can block the binding of PD-L1 to its receptor PD-1, with a blocking ability comparable to that of 12A4 and Atezulizumab analogs.

### Example 10: Flow Cytometry Assay for Humanized PD-L1 Antibody Binding

Human melanoma A375 cells and human lung adenocarcinoma A549 cells were diluted to 1×10⁶ cells/mL with PBS and added to 1.5 mL EP tubes at 100 µL/tube. 10 µL/tube of rat serum was added and blocked at 4°C for 30 minutes. Different concentrations of humanized antibody h19B8-hIgG1LALA (heavy chain and light chains sequences as set forth in SEQ ID NO: 12 to 13) or control antibody 12A4-hIgG1LALA, and Atezulizumab analog (purchased from Bio-Innovation) were added and incubated at 4°C for 30 min. 1 mL of PBS was added to the EP tube. The system was centrifuged at 4°C and 3500 rpm for 5 min. The supernatant was discarded, and the system was washed again with PBS. After centrifugation, the supernatant was discarded, and the cells were resuspended with 100 µL/tube PBS. 1 µL/tube of Alexa-647-labeled rat anti-human secondary antibody (Biolegend) was added and incubated at 4°C in the dark for 30 min. The system was washed twice with PBS, and the supernatant was discarded after centrifugation. The cells were resuspended with 200 µL/tube PBS and detected by flow cytometry. The results are shown in FIG. 8 and further demonstrate that the chimeric antibody of the present disclosure can bind to the PD-L1 protein on the surface of tumor cells, and its binding is stronger than that of the control antibody 12A4 and the Atezulizumab analog.

### Example 11: Humanized PD-L1 Antibody Promotes SEB-Induced IL-2 Secretion by Human PBMCs

Human PBMCs were diluted to 2×10⁶ cells/mL with complete RPMI-1640 medium and added to a 96-well plate at 100 µL/well. 50 µL/well of different concentrations of humanized antibodies h19B8-hIgG1LALA or Atezulizumab analogs were added and blocked at 4°C for 30 min. Different concentrations of humanized antibodies h19B8-hIgG1LALA (as set forth in SEQ ID NO: 12 to 13) or control antibody Atezulizumab analogs (purchased from Bio-Tech) were added. 50 µL/well of 0.4 µg/ml SEB was added, and the system was cultured in a 37°C, 5% CO₂ incubator for 72 h. The IL-2 content in the culture supernatant was detected using a human IL-2 ELISA detection kit. The results are shown in FIG. 9 and further demonstrate that the humanized PD-L1 antibody of the present disclosure can promote IL-2 secretion by PBMCs (wherein the PBMCs in FIG. A and FIG. B are from different human individuals), and the promoting ability is comparable to or slightly superior to that of the Atezulizumab analog.

### Example 12: ELISA Assay of CD3×PD-L1 Antibody Binding

The ELISA assay was used to detect the binding characteristics of the PD-L1 antibody. The PD-L1 extracellular region fusion protein was coated into a 96-well plate. The signal intensity after antibody addition was used to determine the binding characteristic of the antibody to PD-L1.

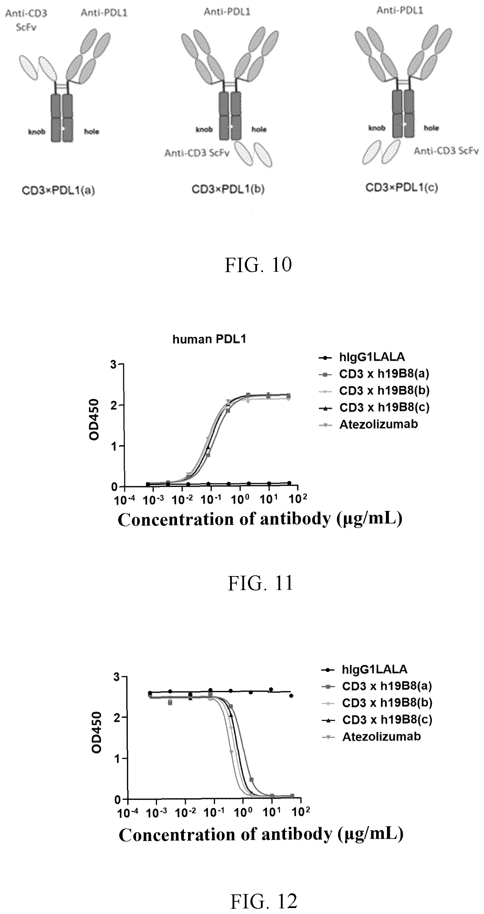

The PD-L1-Fc fusion protein was diluted to 1 µg/ml with PBS buffer, added to a 96-well plate at 100 µl/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, the humanized antibody h19B8-hIgG1LALA and the bispecific antibody CD3×h19B8 to be tested diluted to an appropriate concentration with PBST/0.05% BSA were added at 100 µl/well and incubated at 37°C for 1 h. The reaction system was removed, and the plate was washed 6 times with PBST. HRP (horseradish peroxidase)-labeled anti-human IgG-Fab secondary antibody was diluted with PBST/0.05% BSA at 100 µl/well and incubated at 37°C for 1 h. After washing the plate 6 times with PBST, 80 µl/well TMB (tetramethylbenzidine) was added and incubated at room temperature for 3 min. 4 M sulfuric acid was added at 80 µL/well to terminate the reaction. The absorbance was read at 450 nm using a microplate reader. FIG. 10 shows several connection modes of the CD3×PD-L1 bispecific antibody of the present disclosure. FIG. 11 shows that the CD3×h19B8 antibody of the present disclosure can bind to the PD-L1 protein, with a binding ability slightly weaker than that of the h19B8 monoclonal antibody.

The specific sequence information of the CD3×h19B8 antibody (schematic diagram shown in FIG. 10) and the CD3×12A4 antibody used in the examples of the present disclosure is shown in Table 2.

**[Table 2]**

| Name of bsAb | Heavy chain 1 | Heavy chain 2 | Light chain 1 |
|---|---|---|---|
| CD3×h19B8(a) | CD3-hIgG1LALAkbss SEQ ID NO: 24 | h19B8-hIgG1LALAhoss SEQ ID NO:23 | h19B8-hK SEQ ID NO: 13 |
| CD3×h19B8(b) | h19B8-hIgG1LALAkbssCross3 SEQ ID NO: 26 | h19B8-hIgG1LALAhoss SEQ ID NO:23 | h19B8-hK SEQ ID NO: 13 |
| CD3×h19B8(c) | h19B8-hIgG1LALAkbss SEQ ID NO: 25 | h19B8-hIgG1LALAhossCross3 SEQ ID NO:27 | h19B8-hK SEQ ID NO: 13 |
| CD3×12A4(a) | CD3-hIgG1LALAkbss SEQ ID NO: 24 | 12A4-hIgG1LALAhoss SEQ ID NO:29 | 12A4-hK SEQ ID NO:30 |

### Example 13: Detection Assay for Blocking Activity of CD3×PD-L1 Antibody

The ELISA assay was used to detect the effect of the antibody on the binding of PD-L1 to its receptor PD-1. The specific assay was as follows.

PD-L1-Fc fusion protein (purchased from Acro) was diluted to 5 µg/mL with PBS buffer, added to a 96-well plate at 100 µL/well, and placed at 4°C overnight. The PBS buffer in the 96-well plate was removed, and the plate was washed 6 times with PBST (pH 7.2 PBS containing 0.1% Tween 20) buffer. PBS/10% BSA was added at 200 µl/well and incubated at 37°C for 2 h for blocking. The blocking solution was removed, and the plate was washed 6 times with PBST. Then, the humanized antibody h19B8-hIgG1LALA or bispecific antibody CD3×h19B8 to be tested diluted to an appropriate concentration with PBST/0.05% BSA and 2 µg/mL PD-1-Avi tag (purchased from Acro) were added at 100 µL/well and incubated at 37°C for 1 h. The reaction system was removed, and the plate was washed 6 times with PBST. HRP (horseradish peroxidase)-labeled Strreptavidin secondary antibody (purchased from Southern biotech) was diluted with PBST/0.05% BSA at 100 µL/well and incubated at 37°C for 1 h. After washing the plate 6 times with PBST, 80 µL/well TMB (tetramethylbenzidine) was added and incubated at room temperature for 3 min. 4 M sulfuric acid was added at 80 µL/well to terminate the reaction. The absorbance was read at 450 nm using a microplate reader. The results are shown in FIG. 12 and indicate that the bispecific antibody CD3×h19B8 of the present disclosure can block the binding of PD-L1 to its receptor PD-1, with a blocking ability only slightly weaker than that of the control Atezulizumab.

### Example 14: CD3×PD-L1 Antibody Promotes Killing of Tumor Cells by PBMCs

This example detected the killing effect of PBMCs using the bispecific antibodies.
(1) Complete RPMI-1640 medium was added to a 16-well RTCA plate at 50 µL/well followed by calibration on the machine.
(2) Human lung adenocarcinoma A375 cells were diluted to 2×10⁵ cells/mL with complete RPMI-1640 medium and added to the RTCA plate obtained in step (1) at 50 µL/well. Then the xCELLigence RTCA MP device was used to detect the cell coefficient for 24 hours at 37°C and 5% CO₂.
(3) The bispecific antibody was diluted into a series of concentration gradients using the complete RPMI-1640 medium and added to the RTCA plate obtained in step (2) at 20 µL/well.
(4) PBMCs (Saily Biotechnology) were diluted to 1.25×10⁶ cells/mL with complete RPMI-1640 medium and added to the RTCA plate obtained in step (3) at 80 µL/well.
(5) The reaction system obtained in step (4) was detected for the cell coefficient at 37°C and 5% CO₂ for 24 hours using the xCELLigence RTCA MP device.

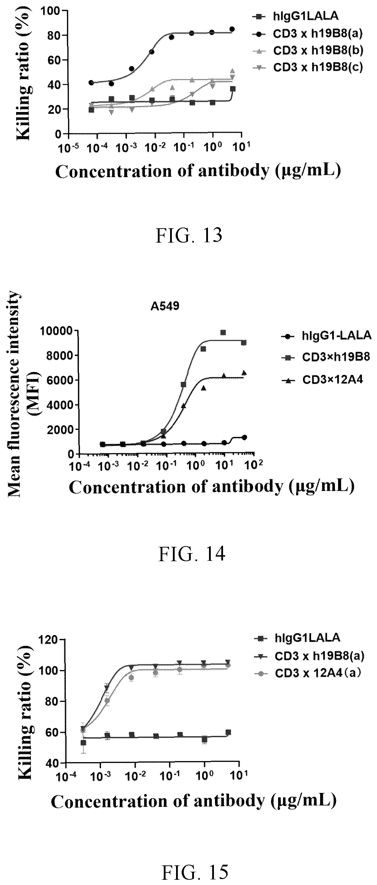

The specific assay results are shown in FIG. 13 and further demonstrate that the CD3×h19B8 (a) bispecific antibody of the present disclosure can promote PBMCs to kill A375 tumor cells, with a killing-promoting ability better than that of (b) and (c) configurations.

### Example 15: Flow Cytometry Assay of CD3×PD-L1 Antibody Binding

Human lung adenocarcinoma A549 cells were diluted to 1×10⁶ cells/mL with PBS and added to a 1.5 mL EP tube at 100 µL/tube. 10 µL/tube of rat serum was added and blocked at 4°C for 30 min. Different concentrations of bispecific antibody CD3×h19B8 or control antibody CD3×12A4 were added and incubated at 4°C for 30 min. 1 mL of PBS was added to the EP tube. The mixture was centrifuged at 4°C and 3500 rpm for 5 min, the supernatant was discarded, and the system was washed again with PBS. After centrifugation, the supernatant was discarded, and the cells were resuspended with 100 µL/tube PBS. 1 µL/tube of Alexa-647-labeled rat anti-human secondary antibody (Biolegend) was added and incubated at 4°C in the dark for 30 min. The system was washed twice with PBS, and the supernatant was discarded after centrifugation. The cells were resuspended with 200 µL/tube PBS and detected by flow cytometry. The results are shown in FIG. 14 and further show that the bispecific antibody CD3×h19B8 of the present invention can bind to the PD-L1 protein on the surface of tumor cells, and its binding is stronger than that of the control antibody CD3×12A4.

### Example 16: CD3×PD-L1 Antibody Promotes Killing of Tumor Cells by PBMCs

This example detected the killing effect of PBMCs using the bispecific antibodies.
(1) Complete RPMI-1640 medium was added to a 16-well RTCA plate at 50 µL/well followed by calibration on the machine.
(2) Human lung adenocarcinoma A375 cells were diluted to 2×10⁵ cells/mL with complete RPMI-1640 medium and add to the RTCA plate obtained in step (1) at 50 µL/well. Then the xCELLigence RTCA MP device was used to detect the cell coefficient for 24 hours at 37°C and 5% CO₂.
(3) The bispecific antibody was diluted into a series of concentration gradients using the complete RPMI-1640 medium and added to the RTCA plate obtained in step (2) at 20 µL/well.
(4) PBMCs (Saily Biotechnology) were diluted to 1.25×10⁶ cells/mL with complete RPMI-1640 medium and added to the RTCA plate obtained in step (3) at 80 µL/well.
(5) The reaction system obtained in step (4) was detected for the cell coefficient at 37°C and 5% CO₂ for 24 hours using the xCELLigence RTCA MP device.

The specific assay results are shown in FIG. 15 and further demonstrate that the CD3×h19B8 bispecific antibody of the present disclosure can promote PBMCs to kill A375 tumor cells, with a killing-promoting ability comparable to or slightly superior to that of the control antibody CD3×12A4.

### Example 17: Anti-cancer Effect of CD3×PD-L1 Antibody in Mice

The in vivo efficacy assay was used to detect the anti-cancer function of the bispecific antibody CD3×h19B8 in improving immune reconstructed mice.
(1) On day -4, human PBMCs were transfused into NCG mice (purchased from GemPharmatech) via the tail vein at 1×10⁷ cells per mouse.
(2) On day 0, NCG mice were subcutaneously injected with 1×10⁶ human melanoma A375 cells per mouse on the right flank.
(2) On days 8, 11, 14, and 17, the mice were injected with the CD3×h19B8(a) antibody via the tail vein, 25 µg per mouse.
(3) The tumor volume was measured every 3 days after injection of the above antibodies.

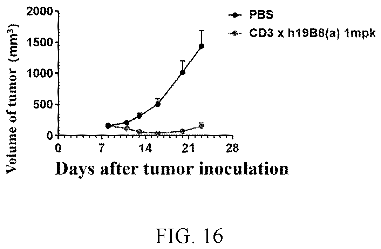

The results are shown in FIG. 16, and it can be seen that the CD3×h19B8 bispecific antibody of the present disclosure can effectively inhibit tumor growth and has anti-cancer function.

It can be seen from the above assay results that the PD-L1 antibody of the present disclosure has the following characteristics: exhibiting stronger binding ability with tumor cells; capable of binding to both human and monkey PD-L1; capable of blocking the binding of PD-L1 to its receptor PD-1; and capable of promoting the anti-cancer function of immune cells.

The present disclosure provides a PD-L1 antibody (h19B8), which binds more strongly to PD-L1 on the surface of tumor cells than antibodies 12A4 and Atezolizumab. Moreover, when recombinantly expressed as a CD3×PD-L1 bispecific antibody, the CD3×h19B8 antibody of the present disclosure also has a stronger binding ability than the CD3×12A4 antibody, suggesting that the bispecific antibody has stronger tumor recognition and thus induces stronger T cell killing. The present disclosure also compares the functional activities of CD3×PD-L1 antibodies with different structures and obtains bispecific antibodies with stronger functional activities.

In the specification, the description of the reference terms such as "one embodiment", "some embodiments", "example", "specific example", or "some examples" means that the specific features, structures, materials, or characteristics described with reference to the embodiment or example are included in at least an embodiment or example of the present disclosure. In this specification, exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine different embodiments or examples and features of different embodiments or examples described in this specification, unless they are contradictory to each other.

Although embodiments of the present disclosure are illustrated and described above, it can be understood that the above embodiments are illustrative and should not be construed as limitations of the present disclosure. Those skilled in the art can make changes, modifications, substitutions, and variations to the above embodiments within the scope of the present disclosure.

## Claims

1. An antibody or antigen binding fragment, comprising a CDR selected from at least one of the following sequences or amino acid sequences having at least 80% identity thereto:
heavy chain variable region CDR sequences: SEQ ID NO: 1, SEQ ID NO: 2, or SEQ ID NO: 3; or
light chain variable region CDR sequences: SEQ ID NO: 4, WAS, or SEQ ID NO: 5.

2. The antibody or antigen binding fragment according to claim 1, comprising:
heavy chain variable region CDR1 with a sequence as set forth in SEQ ID NO: 1,
heavy chain variable region CDR2 with a sequence as set forth in SEQ ID NO: 2,
heavy chain variable region CDR3 with a sequence as set forth in SEQ ID NO: 3,
light chain variable region CDR1 with a sequence as set forth in SEQ ID NO: 4,
light chain variable region CDR2 with a sequence of WAS, and
light chain variable region CDR3 with a sequence as set forth in SEQ ID NO: 5.

3. The antibody or antigen binding fragment according to claim 1, comprising at least one of:
(a) a heavy chain variable region with a sequence as set forth in SEQ ID NO: 6 and a light chain variable region with a sequence as set forth in SEQ ID NO: 7; or
an amino acid sequence having at least 80% sequence identity to (a).

4. The antibody or antigen binding fragment according to claim 1, comprising at least one of:
(b) a heavy chain variable region with a sequence as set forth in SEQ ID NO: 8 and a light chain variable region with a sequence as set forth in SEQ ID NO: 9; or
an amino acid sequence having at least 80% sequence identity to (b).

5. The antibody or antigen binding fragment according to claim 1, comprising:
a heavy chain variable region with a sequence as set forth in SEQ ID NO: 6 or SEQ ID NO: 8; and/or
a light chain variable region with a sequence as set forth in SEQ ID NO: 7 or SEQ ID NO: 9.

6. The antibody or antigen binding fragment according to claim 5, comprising:
a heavy chain variable region with a sequence as set forth in SEQ ID NO: 6 and a light chain variable region with a sequence as set forth in SEQ ID NO: 7; or
a heavy chain variable region with a sequence as set forth in SEQ ID NO: 8 and a light chain variable region with a sequence as set forth in SEQ ID NO: 9.

7. The antibody or antigen binding fragment according to any one of claims 1 to 6, wherein:
the antibody or antigen binding fragment comprises at least one of a heavy chain constant region and a light chain constant region, and wherein
at least a portion of the at least one of the heavy chain constant region and the light chain constant region is derived from at least one of a primate antibody and a murine antibody or a mutant thereof.

8. The antibody or antigen binding fragment according to any one of claims 1 to 7, wherein the light chain constant region and the heavy chain constant region are both derived from a murine IgG antibody or a mutant thereof or a human IgG antibody or a mutant thereof.

9. The antibody or antigen binding fragment according to any one of claims 1 to 7, wherein the light chain constant region and the heavy chain constant region are both derived from a murine IgG1 antibody or a mutant thereof or a human IgG1 antibody or a mutant thereof.

10. The antibody or antigen binding fragment according to any one of claims 1 to 8, wherein:
the N-terminus of the heavy chain constant region is linked to the C-terminus of the heavy chain variable region, and
the N-terminus of the light chain constant region is linked to the C-terminus of the light chain variable region.

11. The antibody or antigen binding fragment according to any one of claims 1 to 10, wherein the antibody or antigen binding fragment has a heavy chain with an amino acid sequence as set forth in SEQ ID NO: 10 or SEQ ID NO: 12 and a light chain with an amino acid sequence as set forth in SEQ ID NO: 11 or SEQ ID NO: 13.

12. The antibody or antigen binding fragment according to any one of claims 1 to 11, wherein:
the antibody or antigen binding fragment has a heavy chain with an amino acid sequence as set forth in SEQ ID NO: 10 and a light chain with an amino acid sequence as set forth in SEQ ID NO: 11; or
the antibody or antigen binding fragment has a heavy chain with an amino acid sequence as set forth in SEQ ID NO: 12 and a light chain with an amino acid sequence as set forth in SEQ ID NO: 13.

13. The antibody or antigen binding fragment according to any one of claims 1 to 12, wherein the antibody or antigen binding fragment comprises a monoclonal antibody or a polyclonal antibody.

14. The antibody or antigen binding fragment according to any one of claims 1 to 13, wherein the monoclonal antibody comprises at least one of a full-length antibody, Fv, a single-chain antibody, Fab, a single-domain antibody, and a minimal recognition unit.

15. The antibody or antigen binding fragment according to any one of claims 1 to 13, wherein the antibody or antigen binding fragment is capable of binding to an amino acid sequence as set forth in SEQ ID NO: 14.

16. A bispecific binding molecule, comprising:
a first binding region, comprising the antibody or antigen binding fragment according to any one of claims 1 to 15; and
a second binding region having a CD3 binding activity.

17. The bispecific binding molecule according to claim 16, wherein the bispecific binding molecule comprises a symmetric bispecific binding molecule or an asymmetric bispecific binding molecule.

18. The bispecific binding molecule according to claim 16 or 17, wherein the bispecific binding molecule is an asymmetric bispecific binding molecule.

19. The bispecific binding molecule according to any one of claims 16 to 18, wherein the first binding region comprises:
peptide chain 1, comprising the heavy chain variable region according to any one of claims 1 to 15; and
peptide chain 2, comprising the light chain variable region according to any one of claims 1 to 15.

20. The bispecific binding molecule according to any one of claims 16 to 19, wherein the second binding region comprises at least one of a full-length antibody, Fv, a single-chain antibody, Fab, a single-domain antibody, and a minimal recognition unit having a CD3 binding activity.

21. The bispecific binding molecule according to any one of claims 16 to 20, wherein the second binding region comprises an anti-CD3 single-chain antibody.

22. The bispecific binding molecule according to any one of claims 16 to 21, wherein the anti-CD3 single-chain antibody comprises:
an anti-CD3 antibody heavy chain variable region, comprising heavy chain variable region CDR1 with a sequence as set forth in SEQ ID NO: 15, heavy chain variable region CDR2 with a sequence as set forth in SEQ ID NO: 16, and heavy chain variable region CDR3 with a sequence as set forth in SEQ ID NO: 17; and
an anti-CD3 antibody light chain variable region, comprising light chain variable region CDR1 with a sequence as set forth in SEQ ID NO: 18, light chain variable region CDR2 with a sequence as set forth in GTN, and light chain variable region CDR3 with a sequence as set forth in SEQ ID NO: 19.

23. The bispecific binding molecule according to any one of claims 16 to 22, wherein the anti-CD3 single-chain antibody comprises:
a heavy chain variable region with a sequence as set forth in SEQ ID NO: 20; and
a light chain variable region with a sequence as set forth in SEQ ID NO: 21.

24. The bispecific binding molecule according to any one of claims 16 to 23, wherein the anti-CD3 single-chain antibody further comprises a linking peptide, and wherein:
the N-terminus of the linking peptide is linked to the C-terminus of the anti-CD3 antibody heavy chain variable region, and the C-terminus of the linking peptide is linked to the N-terminus of the anti-CD3 antibody light chain variable region; or
the N-terminus of the linking peptide is linked to the C-terminus of the anti-CD3 antibody light chain variable region, and the C-terminus of the linking peptide is linked to the N-terminus of the anti-CD3 antibody heavy chain variable region.

25. The bispecific binding molecule according to any one of claims 16 to 24, wherein the linking peptide has an amino acid sequence of (GGGGS)n, where n is an integer greater than or equal to 1, preferably 1, 2, 3, 4, 5, 6, 7, 8, 9, or 10.

26. The bispecific binding molecule according to any one of claims 16 to 25, wherein the anti-CD3 single-chain antibody has an amino acid sequence as set forth in SEQ ID NO: 22.

27. The bispecific binding molecule according to any one of claims 16 to 26, wherein:
the first binding region further comprises at least one of a first heavy chain constant region and a light chain constant region, and wherein
at least a portion of the at least one of the first heavy chain constant region and the light chain constant region is derived from at least one of a human antibody, a primate antibody, a murine antibody, or a mutant thereof.

28. The bispecific binding molecule according to any one of claims 16 to 27, wherein the first heavy chain constant region and the light chain constant region are both derived from a human IgG antibody or a mutant thereof.

29. The bispecific binding molecule according to claim 16 to 28, wherein the first heavy chain constant region and the light chain constant region are both derived from a human IgG1 antibody or a mutant thereof.

30. The bispecific binding molecule according to any one of claims 16 to 29, wherein:
the N-terminus of the first heavy chain constant region is linked to the C-terminus of the heavy chain variable region, and
the N-terminus of the light chain constant region is linked to the C-terminus of the light chain variable region.

31. The bispecific binding molecule according to any one of claims 16 to 30, wherein:
the peptide chain 1 has an amino acid sequence as set forth in SEQ ID NO: 12, and
the peptide chain 2 has an amino acid sequence as set forth in SEQ ID NO: 13.

32. The bispecific binding molecule according to claims 16 to 31, wherein the peptide chain 1 and the peptide chain 2 are linked via a disulfide bond.

33. The bispecific binding molecule according to any one of claims 16 to 32, wherein:
the second binding region further comprises a second heavy chain constant region, and wherein
at least a portion of the second heavy chain constant region is derived from at least one of a human antibody, a primate antibody, a murine antibody, or a mutant thereof.

34. The bispecific binding molecule according to any one of claims 16 to 33, wherein the second heavy chain constant region is derived from a human IgG antibody or a mutant thereof.

35. The bispecific binding molecule according to any one of claims 16 to 34, wherein the second heavy chain constant region is derived from a human IgG1 antibody or a mutant thereof.

36. The bispecific binding molecule according to any one of claims 16 to 35, wherein the N-terminus of the second heavy chain constant region is linked to the C-terminus of the anti-CD3 single-chain antibody.

37. The bispecific binding molecule according to any one of claims 16 to 36, wherein the first heavy chain constant region and the second heavy chain constant region are linked via a knob-into-hole structure.

38. An isolated polynucleotide, encoding the antibody or antigen binding fragment according to any one of claims 1 to 15 or the bispecific binding molecule according to any one of claims 16 to 37.

39. An expression vector, carrying the polynucleotide according to claim 38.

40. A recombinant cell,
carrying the polynucleotide according to claim 38 or the expression vector according to claim 39,
expressing the antibody or antigen binding fragment according to any one of claims 1 to 15, or
encoding the bispecific binding molecule according to any one of claims 16 to 37.

41. The recombinant cell according to claim 40, wherein the recombinant cell is obtained by introducing the expression vector according to claim 39 into a host cell.

42. The recombinant cell according to claim 41, wherein the recombinant cell is a eukaryotic cell.

43. The recombinant cell according to claim 41, wherein the recombinant cell is a mammalian cell.

44. A composition, comprising:
the antibody or antigen binding fragment according to any one of claims 1 to 15,
the bispecific binding molecule according to any one of claims 16 to 37,
the polynucleotide according to claim 38,
the expression vector according to claim 39, or
the recombinant cell according to any one of claims 40 to 43.

45. A method for preparing the antibody or antigen binding fragment according to any one of claims 1 to 15 or the bispecific binding molecule according to any one of claims 16 to 37, the method comprising culturing the recombinant cell according to any one of claims 40 to 43.

46. A medicament, comprising:
the antibody or antigen binding fragment according to any one of claims 1 to 15,
the bispecific binding molecule according to any one of claims 16 to 37,
the polynucleotide according to claim 38,
the expression vector according to claim 39,
the recombinant cell according to any one of claims 40 to 43, or
the composition according to of claim 44.

47. A kit, comprising:
the antibody or antigen binding fragment according to any one of claims 1 to 15,
the bispecific binding molecule according to any one of claims 16 to 37,
the polynucleotide according to claim 38,
the expression vector according to claim 39, or
the recombinant cell according to any one of claims 40 to 43.

48. Use of the antibody or antigen binding fragment according to any one of claims 1 to 15, the bispecific binding molecule according to any one of claims 16 to 37, the polynucleotide according to claim 38, the expression vector according to claim 39, the recombinant cell according to any one of claims 40 to 43, or the composition according to claim 47 in the manufacture of a medicament for preventing and/or treating a PD-L1-mediated disease.

49. The use according to claim 48, wherein the PD-L1-mediated disease comprises cancer.

50. The use according to claim 49, wherein the cancer comprises at least one selected from:
lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

51. Use of the bispecific binding molecule according to any one of claims 16 to 37, the polynucleotide according to claim 38, the expression vector according to claim 39, the recombinant cell according to any one of claims 40 to 43, or the composition according to claim 47 in the manufacture of a medicament for preventing and/or treating a PD-L1- and CD3-mediated disease.

52. The use according to claim 51, wherein the PD-L1- and CD3-mediated disease comprises cancer.

53. The use according to claim 52, wherein the cancer comprises at least one of:
lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

54. Use of the antibody or antigen binding fragment according to any one of claims 1 to 15, the bispecific binding molecule according to any one of claims 16 to 37, the polynucleotide according to claim 38, the expression vector according to claim 39, or the recombinant cell according to any one of claims 40 to 43 in the manufacture of a kit for detecting PD-L1.

55. Use of the bispecific binding molecule according to any one of claims 16 to 37, the polynucleotide according to claim 38, the expression vector according to claim 39, or the recombinant cell according to any one of claims 40 to 43 in the manufacture of a kit for detecting PD-L1 and/or CD3.

56. Use of the antibody or antigen binding fragment according to any one of claims 1 to 15, the bispecific binding molecule according to any one of claims 16 to 37, the polynucleotide according to claim 38, the expression vector according to claim 39, the recombinant cell according to any one of claims 40 to 43, the composition according to claim 44, or the medicament according to claim 46 in the prevention and/or treatment of a disease, wherein:
the disease comprises cancer, and
the cancer is **characterized by** positive PD-L1 on the surface of cancer cells.

57. The use according to claim 56, wherein the cancer is selected from at least one of: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.

58. A method for preventing or treating a tumor, the method comprising administering to a subject at least one of:
the antibody or antigen binding fragment according to any one of claims 1 to 15,
the bispecific binding molecule according to any one of claims 16 to 37,
the polynucleotide according to claim 38,
the expression vector according to claim 39,
the recombinant cell according to any one of claims 40 to 43,
the composition according to claim 44, or
the medicament according to claim 46.

59. The method according to claim 58, wherein:
the tumor is **characterized by** tumor cells that are positive for surface expression of PD-L1, and
the tumor is selected from at least one of: lung cancer, liver cancer, ovarian cancer, cervical cancer, skin cancer, bladder cancer, colon cancer, breast cancer, glioma, kidney cancer, gastric cancer, esophageal cancer, oral squamous cell carcinoma, or head-and-neck cancer.
